# EUROPEAN PATENT APPLICATION

(11) **EP 3 992 302 A1**
(43) Date of publication of application: **04.05.2022**
(21) Application number: 21205713.7
(22) Date of filing: 29.10.2021
(51) Int. Cl.: C12Q 1/6853, C12Q 1/6858, C12Q 1/6823

(54) **GENERIC CARTRIDGE AND METHOD FOR MULTIPLEX NUCLEIC ACID DETECTION**

(30) Priority: 29.10.2020 EP 20204806; 17.12.2020 EP 20214901
(71) Applicant: Biocartis NV, 2800 Mechelen (BE)
(72) Inventor: DEVOGELAERE, Benoit, 2540 Hove (BE); CLAES, Bart, 9255 Buggenhout (BE)
(74) Representative: Nederlandsch Octrooibureau

(57) **Abstract**

The field of the invention generally relates to detection of nucleic acid targets in a multiplex reaction setting. In particular, disclosed herein are methods, kits, kits of parts, systems or components thereof for performing a multiplex PCR detection using custom genetic target panels in a generic detection cartridge. The disclosed methods and kits can typically be utilized for quickly designing automated multiplex PCR-based detection assays for a large number, i.e. tens or multiples of tens, of personalized and/or customized genetic targets, including mutations, SNPs, pathogenic sequences, epigenetic lesions etc. The general principle underlying the disclosed methods and products is a provision of: (1) a panel-agnostic generic detection cartridge preloaded with generic reporter; and, separately therefrom (2) a target-specific multiplex PCR oligonucleotide pool, which, in the target presence under PCR amplification conditions, leads to generation of a molecule capable of specifically reacting with and generating a signal from the generic reporter inside of the cartridge. Consequently, the disclosed herein methods and products enormously simplify the standard diagnostic assay development pipeline, and are hence highly advantageous for brining custom-selected genetic testing panels to laboratories and patients at a rate faster than ever possible before.

## Description

### TECHNICAL FIELD

The field of the invention generally relates to detection of nucleic acid targets in a multiplex reaction setting. In particular, disclosed herein are methods, kits, kits of parts, systems and components thereof for performing a multiplex PCR detection using custom genetic target panels in a generic detection cartridge. The disclosed methods and kits can typically be utilized for quickly designing automated multiplex PCR-based detection assays for a large number, i.e. tens or multiples of tens, of personalized and/or customized genetic targets, including mutations, SNPs, pathogenic sequences, epigenetic lesions etc. The general principle underlying the disclosed methods and products is a provision of: (1) a panel-agnostic generic detection cartridge preloaded with generic reporter; and, separately therefrom (2) a target-specific multiplex PCR oligonucleotide pool, which, in the target presence under PCR amplification conditions, leads to generation of a molecule capable of specifically reacting with and generating a signal from the generic reporter inside of the cartridge. Consequently, the disclosed herein methods and products enormously simplify the standard diagnostic assay development pipeline, and are hence highly advantageous for bringing custom-selected genetic testing panels to laboratories and patients at a rate faster than ever possible before.

### BACKGROUND

There exists a global need for fast and straight-forward development of personalized diagnostic assays that allow to monitor genetically-versatile disease states like infections, organ transplant rejection or cancer in individuals. Such assays should be based on patient-specific genetic information, provide a high reliability and remain cost-effective. The currently existing methods are too time- and resource-intensive, and consequently fail to provide a suitable solution.

In the cancer field, already since the first years of the 21st century, we are observing a paradigm shift from cancer-type specific diagnosis and treatment to a more individualized approach to diagnosis and pan-cancer treatment with a strong focus on individual's underlying genetics and immune response (Hanahan and Weinberg, 2011, Cell). It is consequently now being more broadly recognized that cancer is not one uniform disease but an umbrella term for many different genetically determined acquired pathological proliferation syndromes, wherein not only every cancer type but even every individual cancer appears to have a unique genetic mutational profile (Ciriello et al., 2013, Nat Gen). Consequently, despite the still very frequent prevalence of detectable and therapeutically-targetable driver mutations such as those in the *KRAS, BRAF,* or *EGFR* genes in many cancers, there exists a substantial number of remaining cancer cases, where individually-focused testing could greatly benefit patients' management and outcomes.

One way of performing such individualized screening is by Next Generation Sequencing (NGS). Despite NGS analyses are gradually becoming more affordable, they are still relatively expensive and require expert involvement for data interpretation, which is usually out of reach for general practitioners. Also, waiting times for obtaining the NGS results are still substantial. Consequently, NGS is, and for a while will likely still remain, a largely unsuitable technique for regular or periodical follow-up cancer care. Given the speed, sensitivity, ease-of-use, and availability of the existing fully automated systems, the possibly best solution could be provided by qPCR-based detection of selected targets. Furthermore, these fully automated systems enable global deployment of the tests, which brings the test closer to the patient and ensures global patient access. Although excellent mutation-panel-specific diagnostic tests are readily available for cancer patients from several providers, including Biocartis NV, with the state of the art assay development pipelines, provision of personalized custom target-specific cartridges is below profitability margin for the manufacturers. Despite the limited funding and return on investment considerations, bringing a new diagnostic cartridge under present development procedures is still associated with the standard development and manufacturing lead times that are too long from an individual's perspective. Consequently, there exists an urgent need to transform cancer follow-up care for individuals by developing personalized-genetic assays much faster and cheaper, especially for molecular surveillance testing, post-surgical and minimal residual disease (MRD) monitoring.

With the sudden hit of global pandemic in 2020, causing lockdowns imposed on many manufacturing companies, which further contributed to extended time-lines and time-to-market periods, and substantially limiting access of many patients to healthcare and disease monitoring systems, it became apparent that a new strategy is needed more than ever for faster and more cost-effective development of custom qPCR-detection-based tests. In particular, in view of the emergence of SARS-CoV-2 as a completely new viral and constantly mutating pathogen, making it challenging to detect, it was particularly desirable for such new strategies to be generally applicable to detection of sequences from other than human organisms and to be extremely customizable and readily adaptable to include detection of rapidly changing sequences.

We believe we have developed such an approach, by providing a new platform comprising a generic, i.e. target-agnostic, general detection cartridge comprising generic reporters and compatible with large-multiplex qPCR target specific customizable oligonucleotide pools that can be introduced to the cartridge with a clinical sample to detect within the cartridge the targets of interest. We believe, that the presented hereby approach will find unprecedented use in personalized oncological monitoring including but not limited to: personalized molecular surveillance testing using liquid biopsies, personalized therapy selections, treatment and/or recurrence monitoring , follow up after surgery, detecting MRD, recurrence monitoring after adjuvant therapy, and even in case of a relapse ,monitoring of acquirement of resistance mutations and response to treatment, as well as in cell therapies, personalized cancer vaccines and neoantigen-targeting immunotherapies. The disclosed herein methods and products are equally applicable to be used in outside cancer applications including transplant monitoring or prenatal testing, as well in detection of non-human sequences, e.g. for detecting viral and bacterial pathogens the field of infectious diseases, detection of sepsis, microbiome characterization and many others.

The present disclosure provides methods, kits, kits of parts, systems, and components thereof, for performing multiplex detection of genetic targets using customized genetic target panels in a generic detection cartridge for a point-of-care (PoC) device. The general principle underlying the disclosed methods and products is based on providing at least the two following separate components:
(1) a panel-agnostic generic detection cartridge preloaded with generic reporters configured to detect a presence of a generic sequence; and, separately therefrom
(2) a target-specific multiplex PCR oligonucleotide pool, which, in the presence of a target under PCR amplification conditions, leads to generation of a molecule (further referred to as a detectable molecule) containing the generic sequences and consequently capable of specifically reacting with and generating a signal from the generic reporter inside of the cartridge.

The above explained concept is very new in the current diagnostic assay development practice. To date, to the applicant knowledge, there only exist assay-specific sample-to-result diagnostic cartridges that come preloaded with appropriate detection chemistries and oligonucleotide reagents specific to the predefined by the assay genetic targets, such as therapy targetable mutations. The existing cartridges can be superbly fast and sensitive in detection of their defined genetic targets, but their designs are associated with substantial efforts in terms of optimization, verification, testing etc. as well as the material cost and waiting times for e.g. target-specific reagents like oligonucleotide probes or other sometimes very elaborate amplification and/or detection systems. Additionally, once a working cartridge with a fixed diagnostic panel is developed, it is not straightforward to further modify or adapt its target specific reagents, e.g. to include additional targets in the panel. For example, one must first exclude any negative interactions between the newly introduced oligonucleotides and the initial ones in order not to hamper the modified product's performance. Additional issues can arise during selection or potential incompatibilities between dyes or quenchers after addition of new target-specific reporters like probes into the existing and previously optimized solutions. The problems can arise on many levels and therefore, one must be aware that an introduction of even a seemingly trivial modification to an existing target-specific assay, in order to make an update or fit a particular user's needs, is not straightforward and may require substantial product redesign efforts.

The presented herein methods and products including kits, kits of parts, cartridges, systems and components, address the above described drawbacks by providing a sample-to-result generic detection cartridge, preloaded with all the necessary sample processing and amplification chemistries, but in place of the diagnostic target-specific reagents of the existing assay-specific cartridges, the generic detection cartridge contains general reporters (e.g. labelled probes) configured to detect the presence of a generic sequence tag. To avoid the design or redesign effort, reduce development costs, and waiting times for the synthesis and delivery of target-specific reporters, such generic detection cartridge can be upfront extensively tested, characterized, produced and stocked for rapid supply to clients such as hospitals, clinics, or testing centers, when needed. The customers can then define and order the desired mixes of custom selected target specific oligonucleotide subsets compatible with said cartridges. An oligonucleotide subset can comprise a target-specific primer or a primer pair, but can also comprise one or more additional oligonucleotides acting as a primer or a probe, depending on the amplification chemistry of choice. The compatibility of the oligonucleotide subset mixes with the generic cartridge, loaded with generic reporters would be dependent on the following: (i) their ability to perform in one multiplex amplification reaction inside of the cartridge; and (ii) configuration of each target-specific oligonucleotide subset to generate, in the presence of its target, a nucleic acid product comprising a generic sequence tag associated with and detectable by a defined generic reporter inside of the cartridge.

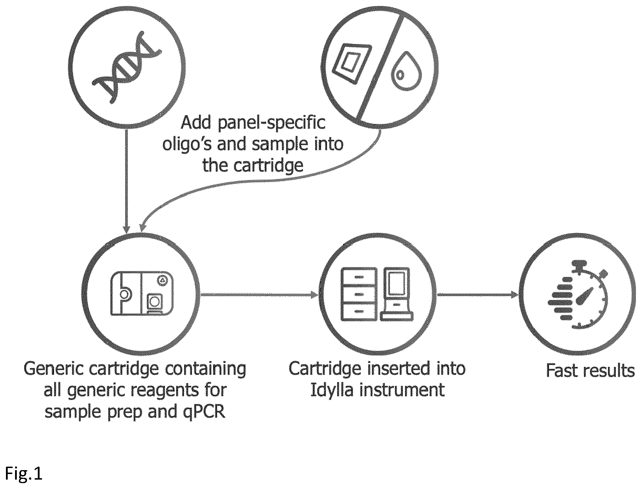

Consequently, from the user perspective, the difference of the above explained concept with regard to the common practice, is that a user receives a two-(or more-)component-product comprising the generic cartridge and the target-specific oligonucleotide pool, instead of a single package with a cartridge already preloaded with reagents specific to a fixed diagnostic panel. Consequently, instead of inserting only a biological sample into the assay-specific cartridge, the user also inserts the mix of target-specific oligonucleotides; a procedure that is schematically illustrated in Fig.1 and constitutes only a minimal additional handling burden vs. the present practice.

Contrary, from the assay design perspective, the generic cartridge-based approach has an enormous potential for substantially shortening the new assay's delivery time by, in our estimation, at least several months to a year, in certain instances up to several years. This is because the nucleic acid isolation chemistry and reporter system can be standardized per generic cartridge type, pushing away the design efforts to concentrate on establishing an efficient multiplexing reaction with the mix of the target specific oligonucleotide subsets.

There is however a broadly established belief in the field that achieving a high level of multiplexing is challenging. A skilled person wanting to detect e.g. 20 different variants e.g. in the currently commercially available Biocartis Idylla^{™} cartridge having 5 amplification chambers, will naturally aim to design five parallel 4-plexes, one in each chamber instead of even considering running one or several in parallel, i.e. each in every chamber, 20-plex reactions. Especially, if a given system has a fixed number of wavelength-specific detection channels associated with amplification chambers and adapted for capturing signals from the reporters therein, it is also unlikely a skilled person would consider running in such amplification chambers a multiplex reaction with a number of targets exceeding the number of detection channels.

The consideration of increasing the number of targets in a multiplex amplification, even becomes more unlikely when low copy targets are concerned. Prominent examples of which include variants present in circulating cell-free DNA (cfDNA) from body fluid samples including plasma or urine. A decent monitoring test should enable the detection of 1% or less, preferably 0.1% or less, cfDNA in a sample from a patient. It is well known to people skilled in the art that the development of a singleplex qPCR assay that can detect 1% variant or lower is already challenging. Consequently, they will know that the development of a multiplex qPCR assay that can detect 1% variant for each of the targeted variants is even more challenging as the different primers and probes used in the qPCR reaction may interfere with each other, thereby reducing the performance of the assay.

Furthermore, the commonly available tools for primer design are still mostly based on rudimentary thermodynamic profiling to predict primer and probe behavior. In reality, however, the reaction conditions typically contain components that were not considered in the thermodynamic model, including buffer additives, enzyme-specific behaviors related to handling of primer-template mismatches, impact of PCR ramp rates and cycle times etc. Consequently, it is also broadly recognized that the available oligonucleotide tools are not particularly suited for predicting primer and probe behavior, which is especially critical for high-performance assays in which less than 1% variant should be detected in a multiplex PCR.

For the above three major reasons relating to the broadly recognized challenges that multiplexing poses, we believe that nobody attempted to date to develop a generic detection cartridge concept as presented herein. However, we have tested the feasibly of the presented herein concept and created an extremely promising generic cartridge prototype compatible with multiplexing custom design genetic target panels introduced thereto with a sample. The underlying design principles of the presently disclosed generic detection cartridge, together with methods, kits, components, and uses, based on it, are generally applicable to all qPCR-based detection strategies, including any random-access sample-to-report devices with one or more PCR chambers. Their advantages include substantial reduction in design effort, material costs, and order waiting times. These and other features and advantages are explained in the continuation.

### SUMMARY

Present disclosure provides methods, kits, kits of parts, systems, and components thereof, for performing multiplex detection of genetic targets using customized genetic target panels in a generic detection cartridge. The general principle underlying the disclosed methods and products is based on providing at least the two following separate components:
(1) a panel-agnostic generic detection cartridge preloaded with generic reporters configured to detect a presence of a generic sequence; and, separately therefrom
(2) a target-specific multiplex PCR oligonucleotide pool, which, in the presence of a target under PCR amplification conditions, leads to generation of a molecule (further referred to as a detectable nucleic acid product) containing the generic sequences and consequently capable of specifically reacting with and generating a signal from the generic reporter inside of the cartridge.

In particular, a first general aspect, a method is disclosed for detecting multiple genetic targets, the method comprising:
- providing a mix of multiple oligonucleotide subsets, each of said subsets being specific to a genetic target and comprising a unique to said subset generic sequence tag,
   wherein each of said subsets is adapted to generate, under nucleic acid amplification conditions and in the presence of the genetic target, a detectable nucleic acid product comprising the unique generic sequence tag;
- separately from the mix, providing an integrated fluidic cartridge comprising
   (i) an entry port for accepting a biological sample,
   (ii) a nucleic acid isolation compartment positioned downstream of the entry port,
   (iii) reagents for nucleic acid amplification;
   (iv) one or more nucleic acid amplification compartments positioned downstream of the nucleic acid extraction chamber, and
   (v) a plurality of generic reporters, wherein each one of the plurality of generic reporters comprises a generic sequence specific to the unique generic sequence tag comprised in one of the detectable nucleic acid products and is adapted to generate a signal in the presence of said detectable nucleic acid product;
      wherein the mix and a biological sample or an extracted nucleic acid are inserted into the cartridge by a user, the method further comprising
- operating the cartridge after the insertion of the biological sample and the mix, wherein the operating comprises performing (inside of the cartridge) nucleic acid isolation from the biological sample followed by a multiplex nucleic acid amplification comprising the mix;
- detecting a signal generated from at least one of the plurality of generic reporters comprised inside of the integrated fluidic cartridge if at least one detectable nucleic acid product is generated from said amplification.

In a further general aspect, kit or a kit of parts is disclosed, said kit comprising, provided as separate components:
- a mix of multiple oligonucleotide subsets, each of said subsets being specific to a genetic target and comprising a unique to said subset unique generic sequence tag, defined as a sequence not present in the genetic information of the organism from which the genetic targets are being detected, wherein each of said subsets is adapted to generate, under nucleic acid amplification conditions and in the presence of the genetic target, a detectable nucleic acid product comprising the unique generic sequence tag; and
- an integrated fluidic cartridge comprising
   (i) an entry port for accepting a biological sample;
   (ii) a nucleic acid isolation compartment positioned downstream of the entry port;
   (iii) reagents for nucleic acid amplification;
   (iv) one or more nucleic acid amplification compartments positioned downstream of the nucleic acid isolation compartment, and
   (v) a plurality of generic reporters, wherein each of the plurality of generic reporters comprises a generic sequence specific to one of the unique generic sequence tags and is adapted to generate a signal in the presence of the detectable nucleic acid product comprising the unique generic sequence tag.

In a yet another general aspect, uses of the methods, kits, components thereof, and their respective embodiments as described in the continuation, are provided for advantageous applications, which for example comprise but are not limited to detecting multiple genetic targets, possibly in a sample obtained from a patient. The patient can be a cancer patient, a patient suffering from an infectious disease, a transplant receiver, or an expecting future mother. If the patient is a cancer patient, advantageous uses of the disclosed methods, kits, and components include but are not limited to post-NGS analysis patient surveillance, response to treatment or therapy monitoring, minimal residual disease (MRD) detection or monitoring, post-surgery follow up, or in individualized cancer neoantigen-targeting immunotherapy selection.

### DESCRIPTION OF FIGURES

For a fuller understanding, reference is made to the following detailed description taken in conjunction with the accompanying figures in which:
**Figure 1****:** shows the general workflow in which all generic reagents (i.e. reagents that are not panel-specific) are present inside a generic cartridge, while the panel-specific reagents and the sample are added by a user through a sample entry port of the generic cartridge;

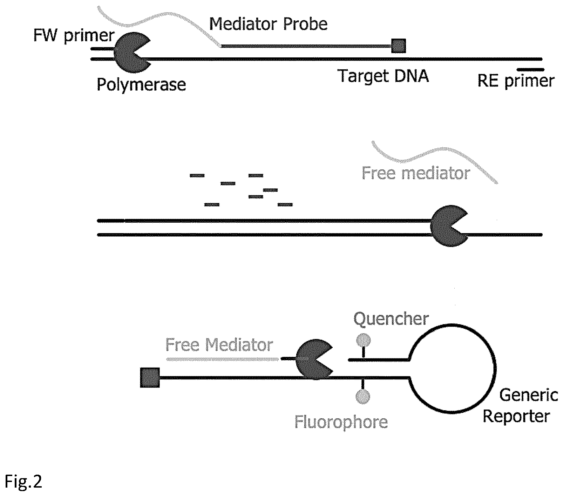

**Figure 2****:** shows the reaction mechanism of the mediator probe PCR. Extension of the forward (FW) primer by a polymerase leads to hydrolysis of the target specific component of the mediator probe and liberation of a free mediator. In a next step, the free mediator can bind to a generic reporter. Note that the fluorophore and quencher can be swapped (not shown). Upon extension of the free mediator, a fluorescent signal is created by displacement of the quencher or fluorophore modification and/or hydrolysis of the quencher or fluorophore-linked nucleotides (not shown). Note that the non-hydrolysed mediator probe and generic reporter cannot be extended by the polymerase (as symbolically indicated by a square);

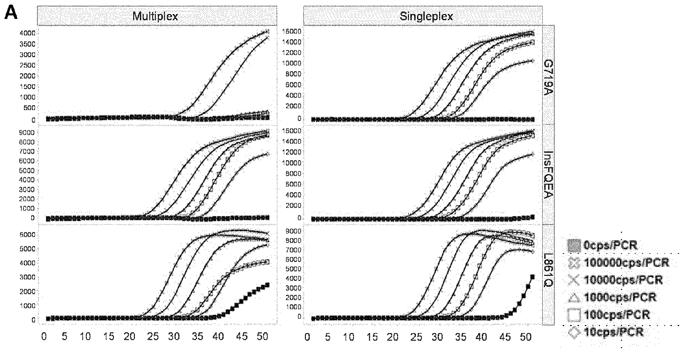

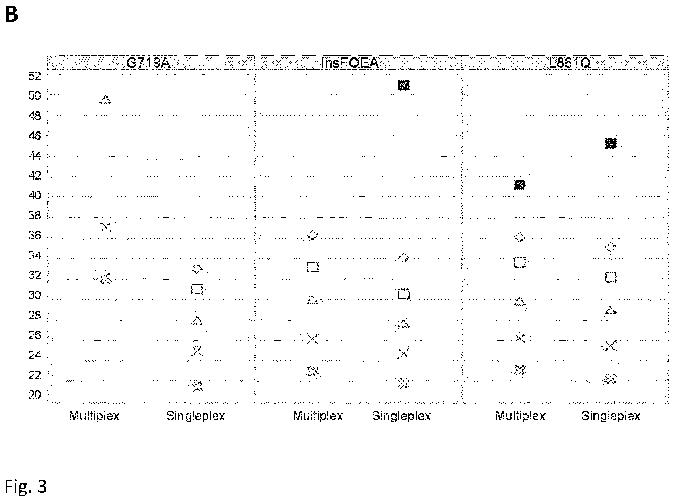

**Figure 3****:** shows the performance for mutation detection using ARMS primers in a singleplex (*i.e.* containing only the primers that are needed to amplify 1 target) as well as in a multiplex (*i.e.* containing primers that are needed to amplify multiple targets) in a 96-well format qPCR instrument. The targets are added as synthetic mutant targets (EGFR G719A; EGFR InsFQEA, EGFR L861Q) at various concentrations in the PCR reaction (which always contains 10,000 copies of genomic wild-type DNA, as well as the oligonucleotide mixture of primers and mediator probes and generic reporters, and the polymerase, dNTPs and PCR salts) as indicated in the legend. Panel A shows the raw curves, panel B shows the Cq values; X-axis: number of PCR cycles, Y-axis: arbitrary fluorescence units;. Solid squares represent the condition where there are 10,000 copies of genomic wild-type DNA present, but no synthetic mutant targets are added.

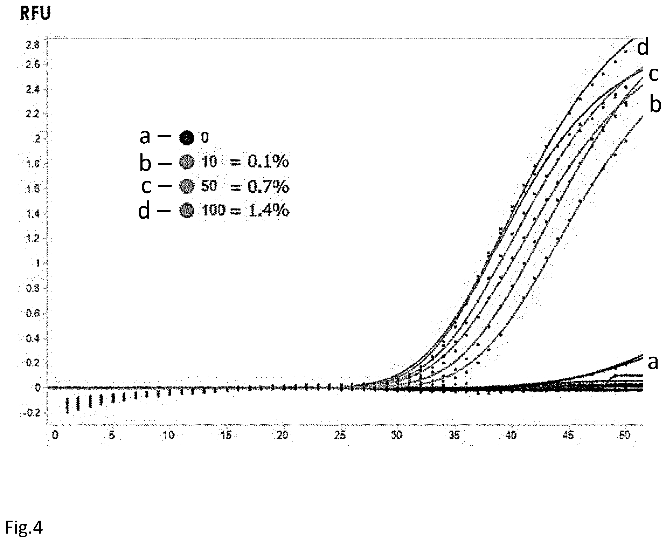

**Figure 4****:** shows the performance for mutation detection using ARMS primers in a multiplex (*i.e.* with an oligonucleotide mixture containing primers that are needed to amplify multiple targets, as well as mediator probes) in an integrated sample-to-result instrument. The targets are added as synthetic mutant targets at various concentrations through the sample entry port, together with the oligonucleotide mixture containing primers and mediator probes, and an formalin-fixed paraffin-embedded (FFPE) clinical sample that was upfront quantified to contain about 7,000 copies of genomic DNA per PCR chamber. The generic reporters, polymerase and dNTPs are spotted in the different chambers of the cartridge. Mutant (synthetic target): 100 (1.4%) - 50 (0.7%) - 10 (0.1%) - 0 (0.0%) copies per PCR. The PCR salts are part of a liquefaction buffer that is generic and present in one of the reagent containers of the cartridge. RFU = Relative Fluorescence Units, *i.e.* the signal obtained from the qPCR component of the integrated sample-to-result instrument;

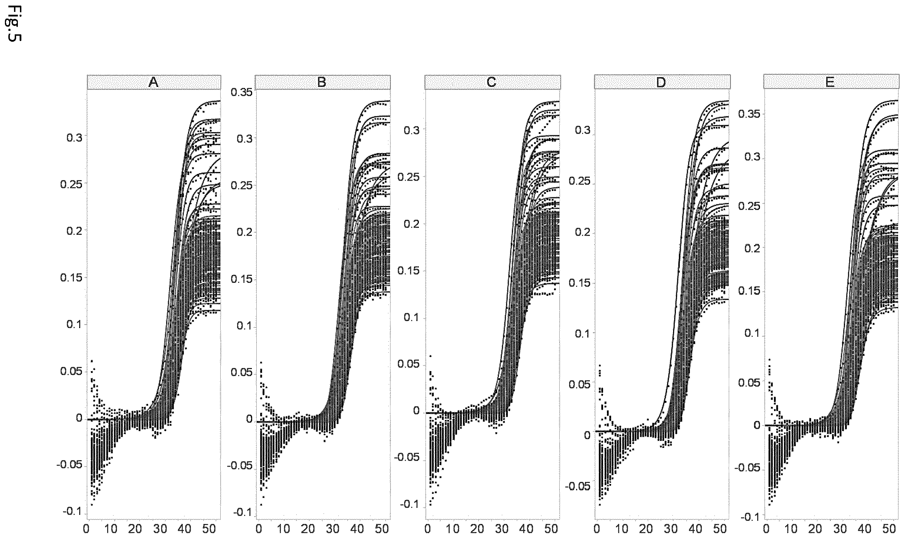

**Figure 5****:** shows the performance for detection of wild-type genomic DNA in a multiplex (*i.e.* with an oligonucleotide mixture containing primers that are needed to amplify multiple targets, as well as mediator probes) in an integrated sample-to-result instrument. The oligonucleotide mixture containing primers and mediator probes, and either an FFPE clinical sample or extracted DNA were added through the sample entry port. The generic reporters, polymerase and dNTPs were spotted in the different chambers of the cartridge. The PCR salts were part of a liquefaction buffer that is generic and present in one of the reagent containers of the cartridge. Y-axis represents signal intensity, X-axis represents cycle number, A-E represents a different PCR compartment;

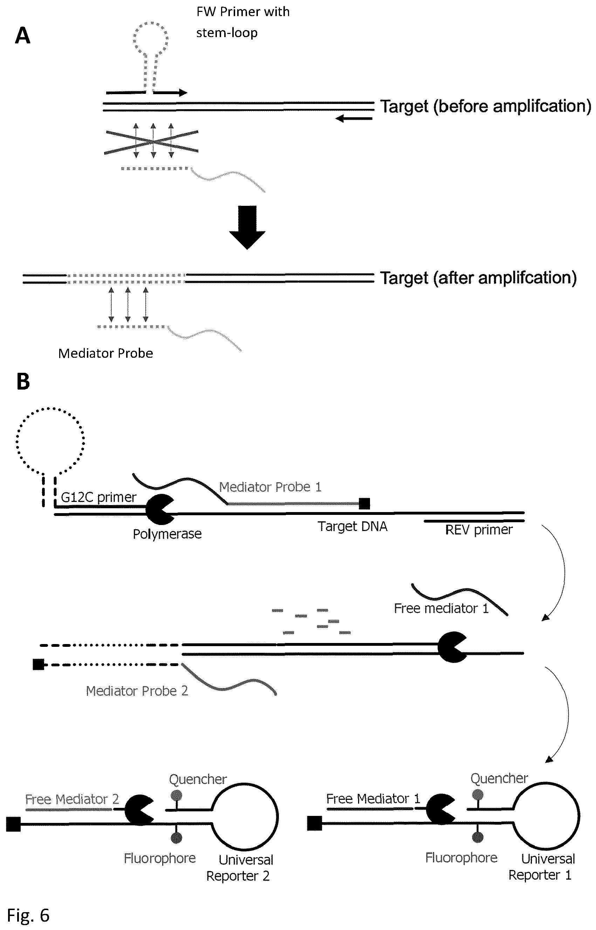

**Figure 6****:** shows concepts enabling the discrimination of neighboring markers. The ARMS primer now includes a stem-loop structure, in which the stem can optionally be composed of the target sequence. Panel A shows one design of an ARMS primer with a stem-loop structure wherein the 5'-end of the primer comprises a sequence specific to the target sequence. The binding site of the mediator probe overlaps (at least partially) with the ARMS primer (indicated as "FW primer with stem-loop). When the target is not amplified, the mediator probe cannot bind to the target sequence and hence cannot create a signal. The mediator probe also cannot bind to the ARMS primer, as it is in a stem-loop configuration and hence inaccessible for the mediator probe. When the target is amplified, the stem structure can be unfolded by the polymerase, thereby creating a binding site for the mediator probe. Once bound, the free mediator is released as the other primer is extended, and the free mediator can bind to the spotted generic reporter and create a signal;
Panel B shows an alternative design an ARMS primer with a stem-loop structure wherein the stem loop-structure defines the 5' end of the primer and can be used as a generic tail tag. Similar to Panel A, the binding site of mediator probe 2 overlaps (at least partially) with the ARMS primer (indicated as "G12C primer). When the target is not amplified, the mediator probe 2 cannot bind to the target sequence and hence cannot create a signal. Mediator probe 2 also cannot bind to the ARMS primer (G12C primer), as it is in a stem-loop configuration and hence inaccessible for the mediator probe. When the target is amplified, the stem structure can be unfolded by the polymerase, thereby creating a binding site for mediator probe. Once bound, the free mediator is released as the other primer is extended, and the free mediator can bind to the spotted generic reporter and create a signal. Presence of mediator probe 1 is facultative, but can be used as a positive control and/or to increase options to detect the target. The mediator probes consist 5' to 3' of a first portion, wherein the first portion comprises a unique generic sequence tag ("UGST") complementary to the UGST binding site of the corresponding generic reporter molecule (universal reporter), a second portion, wherein the second portion is complementary to the target (mediator probe 1) or complementary to the ARMS primer (mediator probe 2) and a polymerase extension blocker (indicated by the square box). The generic reporter molecules (universal reporter) are singled-stranded DNA, and comprise from 5 to 3': i)a first member of a fluorophore/quencher pair; ii) a stem-loop structure; iii) a second member of a fluorophore/quencher pair; iv) a unique generic sequence tag ("UGST") binding site; and v) a polymerase extension blocker.

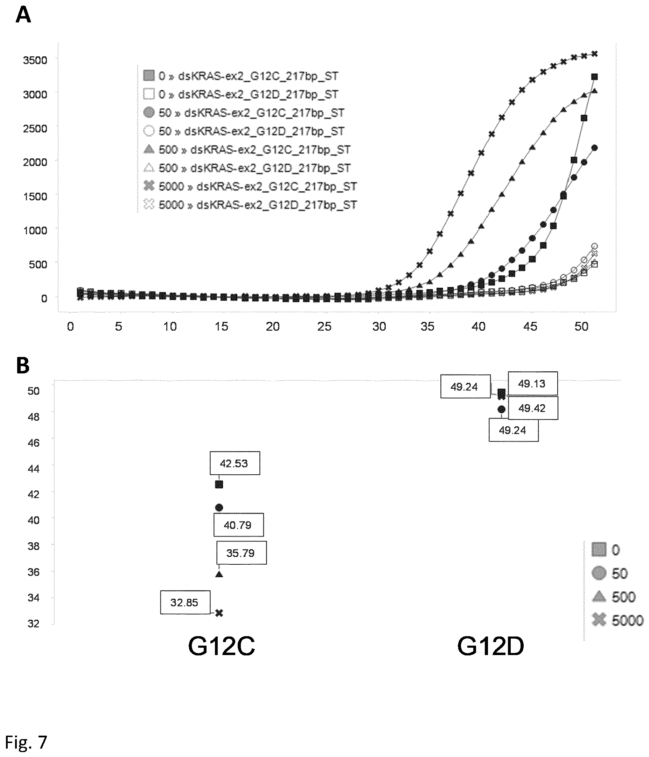

**Figure 7****:** shows the demonstration of discriminating between KRAS G12C from KRAS G12D and wild-type background in accordance with the concept shown in Fig.6. in a multiplex (*i.e.* with an oligonucleotide pool containing primers that are needed to amplify multiple targets, as well as mediator probes). An ARMS primer with a stem loop that enables selective amplification of KRAS G12C is added together with a reverse primer, as well as a mediator probe that is designed to bind to the stem loop proportion of the incorporated KRAS G12C ARMS primer. The mix also contains 10,000 genomic copies of wild-type DNA, along with the polymerase and all other components that are needed to have a functional PCR reaction. Either the synthetic KRAS G12C mutant target (see top panel A, and left part of bottom panel B) or the synthetic KRAS G12D mutant target (see top panel A, and right part of bottom panel B) is added in a 10-fold titration series (with estimated input 5000-500-50-0 copies/PCR) to the mix). The mix is added to different wells of a 96-well qPCR compatible plate. The top part represents the PCR curves, the bottom part shows the Cq values of the same PCR curves. The observed data demonstrate that the stem loop in the ARMS primer enables the discrimination of KRAS G12C from KRAS G12D and wild-type background down to at 500 copies and lower in 10,000 genomic copies of wild-type DNA;

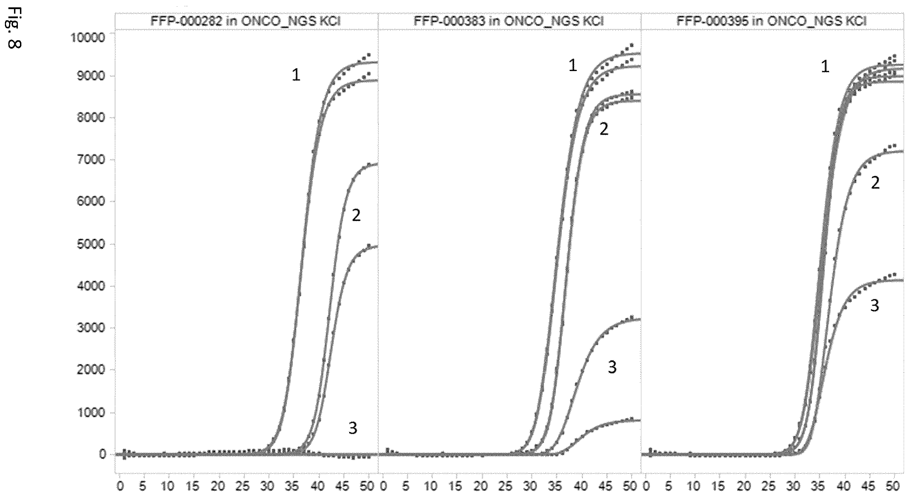

**Figure 8****:** shows how the degree of fragmentation can be verified using KIF11-based QC plex. The righthand panel shows a good quality sample; in this case, all curves (1, 2, & 3) cross the threshold around the same Cq value. In the middle panel, the curves for the middle (2) and longest (3) amplicon shift to the right (i.e., in the direction of higher Cq values). This means that the sample contains less "long" DNA and is therefore fragmented. The left-hand panel represents a very fragmented sample, as can be appreciated by the absence of a qPCR result for the longest amplicon (3) and the shift to higher Cq values for the middle amplicon (2).

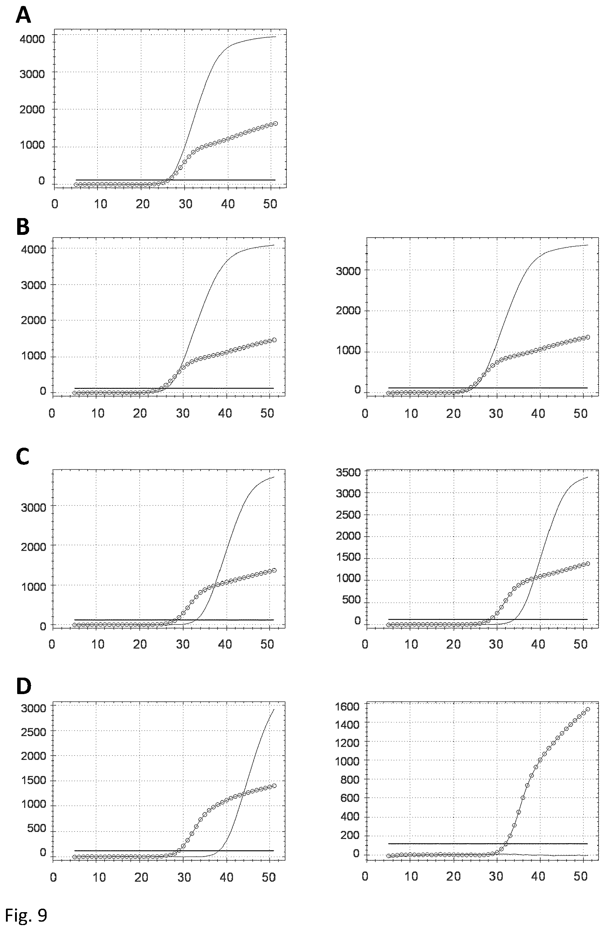

**Figure 9****:** shows that the delta Cq between the long and short KIF11 amplicons as detected by mediator chemistry strongly correlates with the degree of DNA fragmentation in the evaluated FFPE samples. The fitted curves for the short KIF11 amplicon are indicated with lines with circles, while the fitted curves for the long KIF11 amplicon are indicated with regular lines. The panels A-D show as follows: (A) No fragmentation, high quality gDNA sample, Delta Cq (long-short): 0.2; (B) Low fragmentation samples; Delta Cq (long-short) 1.2 (left) and 0.9 (right); (C) Medium fragmentation samples; Delta Cq (long-short) 4.7 (left) and 5.6 (right); (D) High fragmentation samples; Delta Cq (long-short) 9.4 (left) and N/A (long amplicon not detected; right).

### DETAILED DESCRIPTION

Present disclosure provides methods, kits, kits of parts, systems, and components thereof, for performing multiplex detection of genetic targets using customized genetic target panels in a generic detection cartridge. In a first general aspect a method for detecting multiple genetic targets is provided, the method comprising:
- providing a mix of multiple oligonucleotide subsets, each of said subsets being specific to a genetic target and comprising a unique to said subset generic sequence tag,
   wherein each of said subsets is adapted to generate, under nucleic acid amplification conditions and in the presence of the genetic target, a detectable nucleic acid product comprising the unique generic sequence tag;

- separately from the mix, providing an integrated fluidic cartridge comprising
   (i) an entry port for accepting a biological sample,
   (ii) a nucleic acid isolation compartment positioned downstream of the entry port,
   (iii) reagents for nucleic acid amplification;
   (iv) one or more nucleic acid amplification compartments positioned downstream of the nucleic acid extraction chamber, and
   (v) a plurality of generic reporters, wherein each one of the plurality of generic reporters comprises a generic sequence specific to the unique generic sequence tag comprised in one of the detectable nucleic acid products and is adapted to generate a signal in the presence of said detectable nucleic acid product;
      wherein the mix and a biological sample or an isolated nucleic acid are inserted into the cartridge by a user, the method further comprising
- operating the cartridge after the insertion of the biological sample and the mix, wherein the operating comprises performing (inside of the cartridge) nucleic acid isolation from the biological sample followed by a multiplex nucleic acid amplification comprising the mix;
- detecting a signal generated from at least one of the plurality of generic reporters comprised inside of the integrated fluidic cartridge if at least one detectable nucleic acid product is generated from said amplification.

As used herein, the term "genetic target" refers to any gene, transcript, nucleic acid in general, or fragment or forms of any of the above, which can be targeted for detection or investigation by a diagnostic assay. Examples of genetic targets include, but are not limited to genes, sometimes referred to "target genes", gene mutants, particular mutations or short nucleotide polymorphisms (SNPs) within genes, allelic forms, or genetic variants. As used herein, the term "variant" may refer to any genetic variant, i.e. any genetic feature that is known or expected to be different across genetic samples. The term "variant" as used herein can be interpreted as a type of a "genetic target" and can refer to particular mutations, SNPs, or genetic rearrangements, including duplications and deletions. Genetic rearrangements, duplications and deletions may affect small regions, such as regions of one or a few basepairs (bp), or large regions, such as large chromosomal defects stretching over multiple kilobasepairs (kbp). In the present context, the term "variant" will typically refer to a known genetic difference between a tissue that requires monitoring in a subject and a normal tissue and can be treated as a term synonymous to the terms "specific allele", "mutation", "SNP", "variants" in line with their standard meaning as used in the field of molecular biology and biotechnology.

As used herein, the term "oligonucleotide" relates to a relatively short or oligomeric, usually below 200 bp, nucleic acid. Oligonucleotides are frequently synthetic and can comprise various modifications, like modified bases, or be conjugated to various molecules of different functionalities etc. As used herein, the term "oligonucleotide subset" is to be interpreted as a functionally-linked group of oligonucleotides that are specific to a "genetic target". The oligonucleotides in an oligonucleotide subset will normally hybridize, depending on the application, within or around the sequence covering or flanking the genetic target, possibly to enable the genetic target's amplification or detection. A typical target-specific oligonucleotide subset will include at least one primer, likely a primer pair, and possibly also an oligonucleotide probe specific to a genetic target such as a genetic variant. As used herein, the term "multiple" in "multiple oligonucleotide subsets" or "multiple genetic targets" is to be understood as referring to more than one, i.e. a plurality of genetic targets. In the context of a multiplex amplification or a multiplex PCR, the term "multiple" will usually refer to more than 10 or in the range of multiples of 10. Then, the term "mix of multiple oligonucleotide subsets" is to be interpreted as a composition, possibly a solution or at least a partially dried form thereof (e.g. lyophilized) containing the multiple oligonucleotide subsets mixed together. In certain contexts, the term "mix" can also refer a "panel" or a "set" comprising the multiple oligonucleotide subsets.

The term "nucleic acid" and its equivalent "polynucleotide", as used herein is given the regular meaning in the field and refers to a polymer of primarily ribonucleotides or primarily deoxyribonucleotides bound together by phosphodiester linkages between the nucleotide monomers. (Deoxy)nucleotides are phosphorylated forms of (deoxy)nucleosides, which most commonly include adenosine, guanosine, cytidine, thymidine, or uridine. These nucleosides consist of a pentose sugar, being ribose or deoxyribose, and a nitrogenous base ("nucleobase", or simply, "base") being either adenine, guanine (that are purines), cytosine, thymine, or uracil (being pyrimidines). The sequence at which these bases (or their nucleosides, or the nucleotides of the latter) follow in a nucleic acid strand is termed "nucleic acid sequence" and is conventionally given in a so called 5'-end to 3'-end direction referring to chemical orientation of the nucleic acid stand. The "5"' originates from the reference to the 5' carbon of the first (deoxy)ribose ring from which the reading of the nucleic acid sequence begins, and the "3"' originates from the 3' carbon of the last (deoxy)ribose ring on which the reading of the nucleic acids sequence ends. A nucleic acid sequences can e.g. be ATATGCC, which is to be interpreted herein as referring to 5'- ATATGCC - 3' nucleic acid sequence. Under the same convention, the latter sequence will be complementary to the sequence 5' - GGCATAT-3', or simply GGCATAT. Nucleic acids include but are not limited to DNA and RNA, including genomic DNA, mitochondrial DNA or methylated DNA, cDNA, mRNA, rRNA, tRNA, hnRNA, microRNA, IncRNA, siRNA, and various modified versions thereof. Nucleic acids can most commonly be obtained from natural sources like biological samples obtained from different types of organisms. On the other hand, nucleic acids can also be synthesized, recombined, or otherwise produced in any of the known human-devised methods (e.g. PCR)

The term "generic sequence tag" is to be understood as a sequence, usually within the length range of oligonucleotides, not present or possibly present at a low to negligible abundance in the genetic information of the organism from which the genetic targets are being detected. Examples of possible unique sequence tags include but are not limited to nullomers, scrambled synthetic sequences, sequence derived from different/phylogenetically distant organism, Unique Molecular Identifiers etc. In the context of the generic sequence tag being "unique" to (an oligonucleotide) subset (from the mix)" is to be understood that exactly one generic sequence tag corresponds to exactly one "oligonucleotide subset" that is specific to one "genetic target".

As used herein, the term "detectable nucleic acid product" refers to a product or a byproduct from an amplification reaction of a genetic target with the oligonucleotide subset specific to said target. The detectable nucleic acid product is to be understood as being detectable by the virtue of comprising a "unique generic sequence tag" that can be detected by a generic reporter, e.g. being a probe with a label. An example of a detectable nucleic acid product can be an amplicon incorporating the generic sequence tag sequence and generated with a primer or a primer pair forming part of a subset from the multiple oligonucleotide subset. An alternative detectable nucleic acid product can be cleaved or otherwise released part of an amplicon, a primer, or a probe, said released part incorporating in its sequence the generic sequence tag. A specific example of such detectable nucleic acid product is a free mediator being a released by cleavage part of a mediator probe and comprising the generic sequence tag.

As used herein, the term "separately" in the particular contexts of the mix of multiple oligonucleotide subsets being *provided separately* from the cartridge, or the cartridge being *provided separately* from said mix, is to be understood that there is no physical connection present between the mix and the cartridge until the moment a user inserts the mix or a part thereof into the cartridge. For example, the mix can be provided in a liquid form inside of a vial or a tube or any other container. In such instance, a user would open such container and pour or, more likely transfer by means of an e.g. pipette, its contents comprising the mix into the cartridge. Alternatively, the mix can be spotted and/or absorbed onto a solid medium, such as cellulose or a piece of parchment, and provided into the cartridge while bound onto said medium. Other alternatives also exist, including beads, dissolvable tablets, or capsules etc. In any of these events, the mix in the context of the present invention is not physically comprised inside of the cartridge until being transferred thereto by a user before, together, or after also providing into the cartridge the biological material or isolated nucleic acid. In possible instances, the mix and the cartridge can even be provided at different points in time, e.g. when they are sold or shipped on different days to the user.

As used herein, the term "biological sample", or simply "sample", is intended to include a variety of specimen or solutions of biological sources, which contain nucleic acid and/or cellular material, irrespective whether it is freshly obtained from an organism (i.e. fresh tissue sample) or preserved by any method known in the art (e.g. an frozen or an FFPE sample). Examples of biological samples include: cultures of cells such as mammalian cells but also of eukaryotic microorganisms, body fluids, body fluid precipitates, lavage specimen, fine needle aspirates, biopsy samples, tissue samples, cancer cells, other types of cells obtained from a patient, cells from a tissue or *in vitro* cultured cells from an individual being tested and/or treated for disease or infection, or forensic samples. Non-limiting examples of body fluid samples include whole blood, bone marrow, cerebrospinal fluid (CSF), peritoneal fluid, pleural fluid, lymph fluid, serum, plasma, urine, chyle, stool, ejaculate, sputum, nipple aspirate, saliva, swabs specimen, wash or lavage fluid and/or brush specimens.

As used herein, the term "liquid biopsy" or a "liquid biopsy sample" shall be understood as referring to any non-tissue specimen, especially body fluid sample, obtained from a subject. Liquid biopsy sources include but are not limited to blood, plasma, serum, urine, cerebrospinal (CSF) fluid, amniotic fluid, other body fluids such as saliva, sweat, tears, breast milk, semen, stool, pleural fluid, peritoneal fluid or washings etc. Analyzing nucleic acids in liquid biopsy samples can minimize the need for expensive, invasive, and frequently painful tissue and/or tumor biopsies to enable dynamic disease or other physiological state monitoring. For example, in cancer patients, cell-free tumor DNA or RNA extracted from liquid biopsies can potentially be used in detection of mutations, translocations or copy number alterations, and the expression of specific cancer markers. Blood (plasma, serum or whole blood alike) is the most commonly described fluid used in liquid biopsy sample analysis in humans. In cancer patients, blood is the source of circulating tumor cells (CTCs), and cell-free DNA (cfDNA) and cell-free RNA (cfRNA) including circulating tumor DNA (ctDNA) and circulating tumor RNA (ctRNA), respectively, released by tumor tissues, which can be used to detect mutations present in the patients' tumors. The DNA can be methylated or not methylated. Of note, ctDNA comprises however only a tiny fraction of cfDNA present in the blood, which highlights the importance of maximizing sample volumes for nucleic acid analyses in order to detect rare mutations. Furthermore, cfDNA is always of low quality and fragmented to the approximate size of a nucleosome (140 bp to 170 bp). Consequently, for certain cancer types including kidney, prostate, and upper and lower tract urothelial carcinomas, alternative liquid biopsy approaches such as urine may be a richer source of tumor-derived material. Urine also has other unique benefits such as ease of acquisition (does not require trained medical staff), lack of patient discomfort (increased patient compliance), and may have fewer contaminating proteins compared to blood.

Also, as used herein the term "nucleic acid isolation" is to be interpreted as any form of releasing nucleic acids from a biological material to make it available for amplification. Within the ambit of present disclosure, the term can encompass any procedure involving liquefaction of a biological sample or any nucleic acid extraction or purification on a solid support, such as silica.

The term "polymerase chain reaction" or "PCR" is to be understood as referring to the common laboratory nucleic acid amplification technique relying on thermal cycling and the use of at least a primer, typically primer pair, and a DNA polymerase. "Quantitative PCR" or simply "qPCR" is herein given the definition of a laboratory technique based on PCR, which is used to amplify and possibly simultaneously detect or quantify a targeted DNA molecule. In contrast to standard PCR where the product of the reaction is detected at its end, i.e. after thermocycling has finished, the key feature of qPCR is that the DNA product is being detected during thermocycling as the reaction progresses in "real time"; hence, the alternative name of qPCR *"real-time PCR".* There currently exist many different types of qPCRs. For example, when starting with a reverse transcription (RT) step, qPCR can be used to quantify numbers of messenger RNAs and is then called a reverse transcriptase qPCR or an RT-qPCR. As used herein the terms *"quantitative PCR"* or simply *"qPCR"* will be employed with preference over the term *"real-time PCR"* or *"RT-PCR"* in order to avoid confusion with *reverse transcription PCR,* also frequently abbreviated as RT-PCR. Most qPCRs use one of the two most common methods for detecting the product amplification in real-time: (a) intercalation of non-specific fluorescent dyes with any double-stranded DNA, or (2) sequence-specific DNA probes consisting of oligonucleotides that are labelled with a fluorescent reporter which permits detection only after hybridization of the probe with its complementary target sequence. The fluorescent signals generated during thermocycling are detected by an appropriate optical detection system and tracked from the moment they pass the background threshold till the reaction reaches plateau. The copy number of the target sequences can be estimated using either relative or absolute quantification strategy, typically by analyzing the shape of the obtained amplification curve (standard curve strategy) or by determining when the signal rises above some threshold value (often called the Ct value, but sometimes also Cp value or Cq value). In relative quantification, the target nucleic acid levels estimated in a given sample using the Ct or standard curve analysis are expressed as relative to values obtained for the same target in another reference sample, for example, an untreated control sample. Conversely, in absolute quantification the qPCR signal is related to input copy number using a standard curve or can also be calculated according to a more recent digital PCR method. For the moment being, the first strategy is still more prevalent and bases the estimation of the target DNA amount by comparing the obtained values with a previously made standard curve. These and other qPCR quantification strategies are broadly known in the art and their calculation can differ in smaller or greater depending on a given application and a qPCR system.

As used herein, the term "primer" refers to an oligonucleotide, whether occurring naturally as in for example a purified restriction digest or produced synthetically, which is capable of acting as a point of initiation of nucleic acid sequence synthesis when placed under conditions in which synthesis of a primer extension product which is complementary to a nucleic acid strand is induced, i.e. in the presence of different nucleotide triphosphates and a polymerase in an appropriate buffer ("buffer" includes pH, ionic strength, cofactors etc.) and at a suitable temperature. One or more of the nucleotides of the primer can be modified for instance by addition of a methyl group, a biotin or digoxigenin moiety, a fluorescent tag or by using radioactive nucleotides or universal. As used herein the term "variant-specific primer" refers to a primer that specifically binds to a variant sequence. Analogously, the term "variant-specific primer pair" refers to a primer pair that, in a PCR reaction, is intended to produce an amplicon only if the variant is present. A variant-specific primer pair may e.g. include a variant-specific primer, such that amplicons are only generated if the variant is present and the variant-specific primer has a suitable binding place. Alternatively, a variant-specific primer pair may include two primers which only bind in the correct orientation and at a suitable distance if the variant is present. For example, if the variant is a deletion, in the absence of the deletion the distance between the primers of the primer pair may be too large to generate an amplicon in the PCR reaction. In the presence of the deletion, the target-bound primers of the variant-specific primer pair have a correct distance for amplicon generation. The same concept can be applied to gene re-arrangements. A type of a variant-specific primer is a so called "ARMS-primer". ARMS stands for Amplification Refractory Mutation System, which is a frequent application of PCR for identification of point mutations or polymorphisms, in which DNA is amplified by allele specific primers. The ARMS PCR uses a pair of primers, including an ARMS primer and usually a common PCR primer and. The ARMS primer will normally have the following spatial features: (1.) length of usually about 20 to 40 bp; (2.) The nucleotide at the 3' end of the primer is usually complementary to the target nucleotide i.e. G for C or C for G and T for A or A for T. Mismatch at this position can dramatically reduce the amplification. A:G, G:A, and C:C mismatches have the worst effect whereas the other mismatches have varying degrees of effect. For example in a mutation with A-T substitution the ARMS primer for the mutant allele should have the last nucleotide complementary to the nucleotide T i.e. it should have A. The primer for the normal allele at the same position should be complementary to the nucleotide A i.e. it should have T; (3.) possibly, an additional one or more mismatches at one of the last five to ten nucleotides of the ARMS primer to further increase its specificity.

The disclosed methods, kits, kits of parts, systems, or components, relate to a cartridge for an automated system, possibly a PoC system or device. As used herein, the term "cartridge" is to be understood as a self-contained assembly of chambers and/or channels, which is formed as a single object that can be transferred or moved as one fitting inside or outside of a larger instrument that is suitable for accepting or connecting to such cartridge. A cartridge and its instrument can be seen as forming an automated system, further referred to as an automated platform. Some parts contained in the cartridge may be firmly connected whereas others may be flexibly connected and movable with respect to other components of the cartridge. Analogously, as used herein the term "fluidic cartridge" shall be understood as a cartridge including at least one chamber or channel suitable for treating, processing, discharging, or analyzing a fluid, preferably a liquid. An example of such cartridge is given in WO2007004103. Advantageously, a fluidic cartridge can be a microfluidic cartridge. In general, as used herein the terms "fluidic" or sometimes "microfluidic" refers to systems and arrangements dealing with the behavior, control, and manipulation of fluids that are geometrically constrained to a small, typically sub-millimeter-scale in at least one or two dimensions (e.g. width and height or a channel). Such small-volume fluids are moved, mixed, separated or otherwise processed at micro scale requiring small size and low energy consumption. Microfluidic systems include structures such as micro pneumatic systems (pressure sources, liquid pumps, micro valves, etc.) and microfluidic structures for the handling of micro, nano- and picoliter volumes (microfluidic channels, etc.). Exemplary and very suitable in the present context fluidic systems were described in EP1896180, EP1904234, and EP2419705. In line with the above, the term "chamber" is to be understood as any functionally defined compartment of any geometrical shape within a fluidic or microfluidic assembly, defined by at least one wall and comprising the means necessary for performing the function which is attributed to this compartment. Along these lines, "amplification chamber" is to be understood as a compartment within a (micro)fluidic assembly, which suitable for performing and purposefully provided in said assembly in order to perform amplification of nucleic acids. Examples of an amplification chamber include a PCR chamber and a qPCR chamber. In accordance with the above, in alternative embodiments, such cartridges may comprise oligonucleotide generic probes.

As used herein, the term "generic reporter" is to be interpreted as any oligonucleotide probe capable of generating a detectable signal or a change in signal, as a result of its hybridization with a at least partially, preferably substantially, complementary to at least its part, the unique generic sequence tag. In a simplification, a generic reporter can be interpreted as a labelled probe-specific to a generic sequence tag.

In brief, a method is disclosed wherein a user not only inserts a biological sample to a cartridge, but also the separately provided mix of multiple custom target specific oligonucleotide subsets. This is in contrast to the procedure with the existing assay-specific cartridges, wherein the target-specific oligonucleotides are provided inside of the cartridge. In presently disclosed instance, however, in order to enable multiplex nucleic acid amplification in the cartridge, the mix of target-specific oligonucleotide subsets has to be provided into the cartridge by a user, equally as the sample potentially containing the genetic targets of interest as defined by the mix. In an instance where at least one detectable nucleic acid product is generated from said amplification, a signal is generated and can be detected from at least one of the plurality of generic reporters comprised within the cartridge.

The presented herein approach allows for very rapid custom assay design, whereby the genetic target panels can completely be defined by the customers. Achieving this flexibility to design a completely-client defined panel and port it into a standard panel-specific cartridge is simply not possible under the current production pipeline reality of diagnostic cartridge manufacturers. The huge efforts and costs invested into bringing a standard panel-specific cartridge into market for a relatively small number of uses by perhaps a single user, would simply not only be non-profitable but likely would not even bring the return of the investment costs involved. However, within the ambit of the disclosed concept, wherein the major investments are spent on a generic detection cartridge and the customized panel design can later be relatively easily streamlined, possibly with help of an machine learning-powered algorithm, personalized panels for monitoring oncology patients are becoming feasible to come into practice. For example, in an interesting example of the disclosed method, at least one, preferably more, of the multiple oligonucleotide subsets are specific to a genetic target that was identified in a Next Generation Sequencing (NGS) analysis previously performed on a sample from an individual from whom the biological sample was obtained and provided into the cartridge. In such instance, for example, a tumor sample from patient is first analyzed by NGS for determining the key tumor-associated lesions. Based on the result, a custom panel of target-specific oligonucleotide subsets comprising oligonucleotide reagents specific to the selected identified lesions and genes of interest can relatively quickly be designed and produced. From this moment on, the status of the selected lesions and genes can readily and cost-effectively be monitored using the generic detection cartridge, the custom designed panel, and a sample from said patient, for example blood or plasma. Like this, following a surgery or a course of treatment, the patient's tumor status and response is monitored and surveilled on molecular level for any possible instances requiring medical intervention or change of treatment, without the need of repeating the NGS analysis.

In a next example of the disclosed methods, kits, kits of parts, systems, and components, compatible with the previous one, the plurality of generic reporters is immobilized inside of the integrated fluidic cartridge, preferably by being immobilized inside of the one or more nucleic acid amplification compartments. Depending on the design of a given generic cartridge, the immobilization can be done by covalent bonding or affinity interactions as known in the art, which could be useful in generic cartridges based on monolithic or etched chip-like structures with channels under controlled liquid flow. Alternative option involves providing reagents in a matrix-containing spot solution, which can later be dried or freeze-dried to a glass state or similar, not only immobilizing the reagents but also protecting them and stabilizing their storage life. Upon contact with aqueous solutions, such dried matrix becomes hydrated and releases captured therein reagents. In line with this, in an example of the disclosed methods, systems, components, kits, kits of parts, the plurality of generic reporters are immobilized in a spot solution.

If such immobilization is made inside of one or more amplification chambers, other reagents can naturally be included in the spot mix together with the generic reporters, which can include e.g. dNTPs and/or enzymes like polymerases and reverse transcriptases. Depending on stability considerations, one or more spots with different reagents can be deposited in the compartment of choice.

In another possible example, compatible with any of the above examples of disclosed methods, kits, kits of parts, systems, and components, spot solutions are provided e.g. including methylation sensitive enzymes. Along these lines, a methylation sensitive restriction enzyme (MSRE) or a methylation dependent restriction enzyme (MDRE) can be added to the spot solution. When the mixture comprising the isolated DNA and the mix of multiple oligonucleotide subsets enters the PCR chamber, the MSRE/MDRE digest unmethylated/methylated recognition sites when incubated at a certain temperature (20-50°C, typically 30-37°C). In a next step, the mixture of DNA, target specific oligonucleotide subset, and the MSRE/MDRE is heated to temperature between 50-110°C, typically between 70-99°C to inactivate the MSRE/MDRE. At the same time, optionally, a hotstart PCR enzyme can be activated, followed by a typical qPCR cycling protocol. Using this approach, any methylation signature can be easily detected within the same generic detection cartridge.

Spotting a generic reporter and/or other reagents is simple and positively correlates with prolonged shelf life. Depending on the reagent type, different sport solution compositions, or means of immobilization can be used. The spotting or immobilization positions for reagents like MSRE/MDRE can also be made in different compartments or channels, depending on a given cartridge infrastructure, position of heaters etc. For example, they can be also provided upstream of the amplification chamber.

Choice of placing different reagents and running processes in given compartments in general will depend on the internal design of a particular generic cartridge. In a next possible example of the disclosed methods, kits, kits of parts, systems, and components, compatible with any of the previous ones, the multiplex nucleic acid amplification and the generation of the signal from the at least one of the plurality of generic reporters is performed inside of the one or more amplification compartments. Such arrangement allows to monitor the reaction and target detection in real time, as well as places the detectable nucleic acid products generated during the amplification in close vicinity of the generic reporters. Other arrangements using a flow cell and separating the amplification and signal detection can also alternatively be envisaged.

In another possible example of hereby disclosed methods, kits, kits of parts, systems, and components, the nucleic acids isolated from the biological sample and the mix of multiple oligonucleotide subsets can be moved inside of the integrated fluidic cartridge into at least two or more different amplification compartments. This is beneficial in at least two instances. One, if repetitions, like duplicates or triplicates of the same reaction are considered, for example for borderline detectable low copy number targets, Or, two, for automated systems having a defined or a fixed number of wavelength-specific detection channels associated with amplification chambers and adapted for capturing signals from the provided therein reporters. In the latter instance, by separating the nucleic acid and oligonucleotide pool mixture between more than two amplification compartments, one can detect more targets from the multiplex reactions by providing or spotting different generic reporters in different amplification compartments, even though the different generic reporters can be conjugated with a dye detectable in the same channel. Hence, in a possible example of this example, different generic reporters are provided in the different amplification compartments within the same cartridge.

Further in an example of the above example, the signal generated in the presence of the detectable nucleic acid product can be generated from a light-emitting dye and, possibly where the different amplification compartments comprising the different generic reporters comprise a set of the same or at least partially overlapping light-emitting dyes. Along these lines, if we imagine a generic detection cartridge having 3 amplification compartments, each compartment monitored in 4 different channels (e.g. red, yellow, green, and blue), the nucleic acid and oligonucleotide pool mixture would be separated between the 3 compartments. In each of the 3 compartments, the same multiplex amplification would take place and in each compartment 4 different generic reporters could signalize in 4 different channels (the red, yellow, green, and blue) the presence of a detectable nucleic acid product of the amplification. Given the fact that each group of the 4 different generic reporters per compartment can be specific to a different target from the multiplex, 12 different targets from the multiplex can be associated with 12 different detection event over the 3 amplification compartments, each compartment allowing detection in 4 channels. In a possible embodiment of the last two examples, for time-considerations the multiplex nucleic acid amplifications with the mix can be performed simultaneously in each of said two or more amplification compartments.

In another aspect, disclosed methods, kits, kits of parts, systems, and components, are provided wherein one or more of the multiple oligonucleotide subsets comprises at least a primer comprising the unique generic sequence tag, and the detectable nucleic acid product is an amplicon comprising said unique generic sequence tag. In this simple embodiment, the oligonucleotide subsets can merely comprise target-specific primer pairs, wherein one of the primers per such pair comprise, e.g. in a stem-loop-structure the generic sequence tag that is detectable by a generic reporter in the generic detection cartridge. In this instance, the detectable nucleic acid product is the amplicon itself as generated as part of the multiplex amplification.

In a completely different alternative embodiment of disclosed methods, kits, kits of parts, systems, and components, are provided wherein one or more of the multiple oligonucleotide subsets comprises at least one primer and at least one mediator probe, said mediator probe comprising the unique generic sequence tag, and wherein the detectable nucleic acid product is a cleaved free mediator comprising said unique generic sequence tag. This embodiment is based on mediator chemistry principle as disclosed in EP2776585 from Albert Ludwig University of Freiburg, and schematically shown in Fig.2. It has been evaluated to work very efficiently in prototype cartridges as described in the Examples section below. We have further modified the original principle by combining it with ARMS primers, which resulted in improved sensitivities. Hence, in some embodiments of the latter example, the at least one primer is an ARMS primer.

For a better discrimination of closely positioned mutations, certain target-specific ARMS primers were further modified to include a stem-loop structure, in which the stem can optionally be composed of the target sequence. We further modified the mediator probe in such a way that it at least partially overlaps with the stem-loop-modified ARMS primer and the concept is show in Fig.6. In top panel A, the 5' end of the ARMS primer comprises a sequence complementary to the target sequence, whereas in the bottom panel B, the 5' end of the ARMS primer terminates with the stem-loop-stem structure that serves as a generic tail tag, which has advantages for streamlined design of such primers. The 3' component of the stem of the ARMS primer can have a sequence that is derived from the target sequence, or can be another sequence. When the target is not amplified, the mediator probe cannot bind to the target sequence and hence cannot create a signal. The mediator probe also cannot bind to its complementary sequence within the ARMS primer, which is inside of the stem-loop configuration and hence inaccessible for the mediator probe. When the target is amplified, the stem structure can be unfolded by the polymerase, thereby creating a binding site for the mediator probe. Once bound, the free mediator is released as the other primer is extended, and the free mediator can bind to the spotted generic reporter and create a signal. In line with the above, in specific embodiments of the last two examples, the ARMS primer comprises a stem-loop structure, and preferably, the mediator probe sequence further at least partially overlaps with the sequence comprised in said stem-loop structure.

In another example, a method is provided wherein the nucleic acid isolation from the biological sample is performed in the presence of the mix of multiple oligonucleotide subsets, possibly within the nucleic acid extraction compartment. In this particular instance, a user can add the mix of multiple oligonucleotide subsets together with the biological sample into the cartridge, which saves time. Surprising, we have observed it works very well with a nucleic acid isolation protocol comprising liquefaction protocol on the Idylla^{™}-based generic cartridge prototype. Alternatives comprise first providing the mix of multiple oligonucleotide subsets and having it pumped deepener into the internal cartridge space, followed by the biological sample addition once the mix achieves the desired compartment in the cartridge, and only them initiating the nucleic acid isolation protocol. Another alternative may involve including two entry ports within the cartridge, one for the mix and one for the biological sample.

In some examples, methods, uses, kits, kits of parts, systems, and components, are provided the biological sample is a solid tissue sample, possibly a fixed solid tissue sample, preferably a formalin-fixed paraffin-embedded (FFPE) sample. In such instances, the nucleic acid isolation could advantageously comprise or consist of liquefaction of said solid tissue sample.

In alternative examples, methods, uses, kits, kits of parts, systems, and components, are provided, wherein the biological sample is a liquid biopsy sample. In a particular embodiment of said example, methods, uses, kits, kits of parts, systems, and components, are provided are provided wherein the nucleic acid isolation comprises isolation of cell-free nucleic acids. In a further embodiment of the latter embodiments, the nucleic acid isolation could advantageously comprise or consist of nucleic acid extraction, preferably on solid support.

In further examples, methods, uses, kits, kits of parts, systems, and components, are provided wherein at least a part of the oligonucleotide subsets from the mix of multiple oligonucleotide subsets, is designed by a computer-implemented method comprising machine learning or artificial intelligence.

In some examples, methods, uses, kits, kits of parts, systems, or components, are provided, wherein the mix of multiple oligonucleotide subsets comprises a subset specific to a region in the KIF11 gene. In their possible embodiments, said region in the KIF11 gene is used as a genomic reference gene, or alternatively, a KIF11 amplicon generated with said subset is used as a genomic reference gene. Preferred embodiments of present examples concern the human KIF11 gene, but possibly can also concern its other mammalian homologue. In particular examples, the region in KIF11 is located in any of the following exons 6, 8, 18, 21, or exon 21- intron 21 boundary. In further embodiments, methods, uses, kits, kits of parts, systems, or components, are provided, wherein one or more of the multiple oligonucleotide subsets comprises a primer specific to a region in the human KIF11 gene, preferably wherein at least two of the multiple oligonucleotide subsets comprise primers specific to a different region in human KIF11 gene, wherein said primers are designed to generate two KIF11 amplicons of discernably different lengths.

As we identified KIF11 as a surprisingly stable genomic region among majority of cancer types, irrespectively of the disclosed herein methods, uses, kits, kits of parts, systems, or components, use of KIF11 as a genomic reference target or a house-keeping gene reference is hereby in general disclosed for any DNA or RNA amplification reaction, in particular in cancer. In particular examples, the region in KIF11 is located in any of the following exons 6, 8, 18, 21, or exon 21 - intron 21 boundary and are hereby also disclosed as suitable genomic reference targets regions.

In another aspect, kits, kit of parts, systems, or components thereof are disclosed comprising, provided as separate components:
- a mix of multiple oligonucleotide subsets, each of said subsets being specific to a genetic target and comprising a unique to said subset generic sequence tag,
   wherein each of said subsets is adapted to generate, under nucleic acid amplification conditions and in the presence of the genetic target, a detectable nucleic acid product comprising the unique generic sequence tag; and
- an integrated fluidic cartridge comprising:
   (i) an entry port for accepting a biological sample; possibly a second entry port for accepting the mix;
   (ii) a nucleic acid isolation compartment positioned downstream of the entry port, possibly said nucleic acid isolation compartment comprising or arranged to become fluidly connected with at least a reagent for nucleic acid isolation compartment, such as liquefaction buffer inside of the cartridge;
   (iii) reagents for nucleic acid amplification;
   (iv) one or more nucleic acid amplification compartments positioned downstream of the nucleic acid isolation compartment (e.g. nucleic acid extraction chamber), and
   (v) a plurality of generic reporters, wherein each of the plurality of generic reporters comprises a generic sequence specific to one of the unique generic sequence tags (that is unique to an oligonucleotide subset and is comprised in one of the detectable nucleic acid products) and is adapted to generate a signal in the presence of the detectable nucleic acid product comprising the unique generic sequence tag.

In some examples, kits, kit of parts, systems, or components thereof are provided wherein the plurality of generic reporters is immobilized inside of the cartridge.

In further examples, kits, kit of parts, systems, or components thereof are provided wherein an oligonucleotide subset from the mix of multiple oligonucleotide subsets comprises at least a primer and a mediator probe, and preferably wherein the primer is an ARMS primer. In some embodiments, further examples are provided wherein the ARMS primer comprises a stem-loop structure and wherein the mediator probe sequence at least partially overlaps with the sequence comprised in said stem-loop structure.

In some other examples, kits, kit of parts, systems, or components thereof, are provided wherein one or more of the multiple oligonucleotide subsets comprises a primer specific to a region in the human KIF11 gene, preferably wherein at least two of the multiple oligonucleotide subsets comprise primers specific to different regions in the human KIF11 gene, wherein said primers are designed to generate two KIF11 amplicons of discernably different lengths. In possible embodiments, the region in the human KIF11 gene is located in any of the following exons 6, 8, 18, 21, or exon 21-intron 21 boundary.

Last but not least, hereby disclosed are uses of disclosed methods, kits, kit of parts, systems, or components thereof, detecting multiple genetic targets, possibly in a sample from a cancer patient, possibly as part of post-NGS analysis patient surveillance or in minimal residual disease monitoring.

### EXAMPLES

### 1. Identification of a generically applicable genomic reference locus

As the goal of the present application was to provide for a generic platform capable of handling a great variety of custom-designed panels for very versatile and possibly genomically unstable diseases and targets, we have first attempted to identify a robust genomic reference locus or gene. In particular oncological diseases are frequently subject to genomic rearrangements and in our extensive oncological practice, we have tested many different house-keeping loci and believe that for each assay a reference gene or a group thereof is best selected in function of the tumor type. However, given the generic nature of the desired herein application, we have set out to screen for a locus that is minimally affected over a wide range of cancer types by different genetic variations, including point mutations and copy number changes. Our extensive literature and *in silico* analyses of our and others' data allowed us to top rank 100 potentially oncologically "stable" genes. These top ranked potential reference loci were further analyzed for somatic variations in The Cancer Genome Atlas (TCGA) database, queried via the public web portal https://www.cbioportal.org

The analysis allowed for further narrowing the selection to smaller number of targets, as a result of which we have identified and extensively verified that KIF11 appears to be an extremely promising genomic reference gene, very rarely affected by somatic mutations or copy number alterations in the tumor samples we tested or screened to date. The suitability of KIF11 as pan-cancer "stable" reference control could also be confirmed in a great variety of tumors of 228 studies included in the TCGA at the time of our analysis, with a slight exception of 2-7% of prostate cancers (TCGA reported amplifications or deletions, depending on the study) and 9% of nerve sheath tumors (amplifications). The latter analysis appears to confirm the surprising suitability of a previously unreported as a genomic reference gene KIF11 for being used as a generically stable control locus for a very wide variety of genomically unstable samples such as tumor samples. Our finding is even more surprising as KIF11 increased expression has widely been reported in many different cancer types and it has even been proposed to be an oncogene or a potential tumor marker in different cancer types (Daigo et al., 2018 Int J Oncol 52:155-165; Pei et al. 2017 Oncol Lett 6618-6626 https://doi.org/10.3892/ol.2017.7053; Imai et al. 2016 Pathobiology 84:16-24). Despite its reported transcriptional overexpression, based on our investigations and the TCGA-obtained data, KIF11 genomic locus appears as exceptionally stable and only when designing assays for prostate cancers or nerve sheath tumors, additional studies would be recommended to de-risk genomic stability of KIF11 as control region of choice.

To further corroborate our finding, we have extensively tested stability of selected KIF11 regions in a broad range of samples and online databases. In particular, for common genetic variations (SNPs), we particularly focused on intron 6, exons 6, 21, intron-exon boundaries around the latter, and the two longest exons 8 and 18 able to accommodate long amplicons (243 bp and 280bp, respectively).

In conclusion, KIF11 appears to be highly genomically stable in terms of copy number alterations and somatic mutations in nearly all cancer types for which public data is available, as well as, in terms of SNPs for the investigated exons and exon 21-intron 21 boundary. Consequently, KIF11 appears to be a very promising generic control with a high likelihood of providing consistent performance across any global population.

### 2. Selection of multiplex amplification chemistry with generic reporters

For the selection of a proof-of-principle nucleic acid amplification strategy comprising generic reporters, several approaches were evaluated. Examples include, but are not limited to, anisotropic loop hairpin primers as described in WO2020/165180 of Biocartis NV, a possible combination of PASS primers and MNAzyme technologies of SpeeDx Ltd as described e.g. in EP2753717, EP2817421, and EP1948822, or a mediator probe chemistry as described in EP2776585 from Albert Ludwig University of Freiburg, schematically shown in Fig.2.

Bearing in mind the desired goal of performing high-target-number multiplexes, potentially containing several tens or more target-specific primer pairs and possibly probes, we have developed an assay based on the combination of ARMS primers and mediator probe chemistry. To date, the mediator probe chemistry has only been used in combination with standard primers, i.e. primers that are not mutation- or variant-selective and amplify a nucleic acid region irrespective of whether a mutation/SNP of interest is present therein. Consequently, to date, where a variant-specific detection would be of interest, the mediator probe would be designed such to partially overlap with the mutation or SNP of interest. However, we found that said approach required extensive optimization of the mediator probes in order to ensure their robust allele-selectivity. In particular, this would generally not allow reaching sensitivities of 1% or less for the detection of SNPs, making it unsuitable for sensitive mutation detection assays. We therefore modified the mediator probe chemistry-based assay in such a way that ARMS primers are used for the selective amplification of the mutant target molecules, and the mediator probes do not overlap with the mutation of interest. ARMS primers can contain e.g. wobbles and/or loops, which we hypothesized could allow in a complex multiplex setting for fine-tuning sensitivity/specificity outcomes for more challenging targets.

The results showing strong discriminative power of the presented herein approach are shown in Fig.3, illustrating the performance of a 3-plex assay that combines the use of ARMS primers and mediator probes for the highly sensitive detection of SNPs and indels in an EGFR gene. The results show that a titration series of synthetic mutant targets was robustly amplified and clearly detectable in a genomic wild type background of 10.000 copies. The 3-plex assay was able to detect down to 10 mutant target copies with sufficient discrimination from the Limit of Blank (LOB, indicated with a solid square in the figure) for some of the targets of the 3-plex assay.

### 3. Configuration of a prototype generic cartridge

The generic cartridge prototype was prepared based on a fluidic sample-processing cartridge proprietary to Biocartis NV and compatible with their automated molecular testing system Idylla^{™}. The cartridge is manufactured as a single disposable entity containing a sample entry port and multiple internal compartments for reagents and waste that communicate with a fluidic path for sample processing and nucleic acid isolation according to a strategy of choice. The fluidic path terminates in five independent nucleic acid PCR amplification chambers preloaded with amplification reagents and configured to accept a portion of the nucleic acids as isolated in upstream sections of the fluidic path. The amplification chambers are equipped with transparent walls that enable detection of signals generated during nucleic acid amplification such as PCR performed with light-emitting dyes signalizing the presence of a genetic target of interest. Once a sample is provided into the cartridge and the cartridge is fed into the Idylla^{™} system, the entire sample-to-result processing programme is orchestrated in a fully automated manner inside of the system, including biological sample disruption, nucleic acid isolation by means of e.g. either liquefaction or solid phase extraction, followed by target nucleic acid amplification, and signal detection.

For creating the first proof-of-principle generic cartridge prototypes, Idylla^{™} cartridges as described above were loaded with reagents and a buffer optimized for liquefaction of fixed solid tissue samples (e.g. FFPE samples), as described in EP2958997 belonging to Biocartis NV. Next, spot solutions of 5 differently-labeled unique generic reporters together with PCR reagents including dNTPs, Taq polymerase, etc. were spotted and dried in accordance with the manufacturer's protocol in each one of the 5 amplification chambers of the cartridge. The Mg²⁺ and buffering agents required for PCR were integrated in the buffer used for liquefaction.

Separately from the generic cartridge prototype, a mix of 61 target-specific oligonucleotide subsets was designed as follows. Each target-specific oligonucleotide subset of the 61 subsets in the mix contained one forward primer, one reverse primer, and one mediator probe. With the exception of the subset specific to KIF11 as the positive control target, forward primers were typically designed as allele-selective ARMS primers, i.e. primers that bind specifically to one allele of interest. In this example, one specific allele is regarded as a target, even though it may concern the same gene as another allele targeted by another subset in the mix. Consequently, it is possible that different target-allele-specific subsets may share oligonucleotides having the same sequences, like in this example. Namely, the reverse primers and the mediator probes in each subset were mostly non-allele-selective, and rather designed to bind to a region in a gene close to the allele of interest. In this experiment, the mix of 61 target-specific oligonucleotide subsets contained 61 forward primers of different sequences (defining the 61 genetic targets comprising different genes and different alleles of the same gene), 9 different reverse primers, and 9 different mediator probes. This is because several reverse primers and mediator probes were designed to constitute part of subsets specific to a different allelic target in the same gene, the different alleles being discriminated by the specificity of the ARMS forward primers. The cartridge layout is given in columns 3-5 of Table 1 below, and the position in which 9 specific targets can be detected is indicated. Sequences of the oligonucleotides are shown in Table 2. The their concentrations in the mix can be obtained from Biocartis NV upon request, but can be determined by the person skilled in the art.

**Table 1**

| **Target** | **Mediator Probe** | **Generic Reporter** | **Amplification chamber** | **Detection Channel** |
|---|---|---|---|---|
| - | 1 | 1 | A | 1 |
| - | 2 | 2 | B | 1 |
| EGFR L858R | 3 | 3 | C | 1 |
| KRAS G12C | 4 | 4 | D | 1 |
| - | 5 | 5 | E | 1 |
| G719A | 6 | 6 | A | 2 |
| EGFR S768I | 7 | 7 | B | 2 |
| BRAF V600E | 8 | 8 | C | 2 |
| - | 9 | 9 | D | 2 |
| - | 10 | 10 | E | 2 |
| - | 11 | 11 | A | 3 |
| - | 12 | 12 | B | 3 |
| HER2 ex20ins | 13 | 13 | C | 3 |
| - | 14 | 14 | D | 3 |
| - | 15 | 15 | E | 3 |
| EGFR ex19del | 16 | 16 | A | 4 |
| EGFR C797S | 17 | 17 | B | 4 |
| - | 18 | 18 | C | 4 |
| - | 19 | 19 | D | 4 |
| - | 20 | 20 | E | 4 |
| KIF11 control | 21 | 21 | A, B, C, D, E | 5 |

**Table 2**

| SEQ ID NO | oligo name | 5>3 sequence |
|---|---|---|
| 1 | 5EGFR_T21_7 | AAAAGATCAAAGTGCTGGC |
| 2 | 5EGFR_T20_7 | GAATT CAAAAAGATCAAAGT GCAGA |
| 3 | 5EGFR_T19_11 | AATTCAAAAAGAT CAAAGT GCGGT |
| 4 | 5EGFR_T22_2 | CTCTTGAGGATCTTGAAGGATGA |
| 5 | 5EGFR_T23_13 | GCTCTCTTGAGGATCTTGTAGA |
| 6 | 5EGFR_T24_13 | CTCTTGAGGATCTTGATGGC |
| 7 | 5EGFR_T25_11 | CTCTTGAGGATCTTGAAGCATAC |
| 8 | 5EGFR_T26_13 | CTCTTGAGGATCTTGATGGG |
| 9 | 5EGFR_T27_13 | TCTCTTGAGGATCTTGATGGT |
| 10 | 5EGFR_T2_11 | ATTCCCGTCGCTATCAAAACA |
| 11 | 5EGFR_T3_11 | CCGTCGCTATCAAGACATCT |
| 12 | 5EGFR_T6_5 | CCGTCGCTATCAAGGAATCGAA |
| 13 | 5EGFR_T10_5 | CGTCGCTATCAAGGAATCTC |
| 14 | 5EGFR_T4_11 | TCGCTATCAAGGAACCAAC |
| 15 | 5EGFR_T17_10 | CGTCGCTATCAAGGTTCCG |
| 16 | 5EGFR_T11_6 | CCGTCGCTATCAAGGCATC |
| 17 | 5EGFR_T16_8 | GTCGCTATCAAGGAACCGAA |
| 18 | 5EGFR_T5_9 | CGTCGCTATCAAGGAACCATC |
| 19 | 5EGFR_T9_6 | CGTCGCTATCAAGGAAGCA |
| 20 | 5EGFR_T12_2 | CGTCGCTATCAAGGAGCCAAC |
| 21 | 5EGFR_T14_8 | CGTCGCTATCAAGGAATCATC |
| 22 | 5EGFR_T15_22 | TCCCGTCGCTATCAAAATATCT |
| 23 | 5EGFR_T8_6 | TCCCGTCGCTATCAAGTCT |
| 24 | 5EGFR_T13_10 | GTCGCTATCAAGGAACAGAA |
| 25 | 5EGFR_T45_5 | CCGTCGCTATCAAGGCTCC |
| 26 | 5EGFR_T46_7 | CGTCGCTATCAAGGATCCG |
| 27 | 5EGFR_T47_8 | GTCGCTATCAAGGAGCAAT |
| 28 | 5EGFR_S768I_3 | GAAGCCTACGTGATGGGCAT |
| 29 | 5FQEA_1 | CTCCAGGAAGCCTTCCAGGA |
| 30 | 5EGFR_C797S_4 | CTCATGCCCTTCGGCTC |
| 31 | 5EGFR_C797S_3 | GCTCATGCCCTTCGGCA |
| 32 | 5EGFR_T49_11 | GTCAAGATCACAGATTTTGGTCG |
| 33 | 5EGFR_T50_2 | GTCAAGATCACAGATTTTGGGCGT |
| 34 | 5EGFR_T51_3 | GATTTTGGGCTGGCCAAACA |
| 35 | 5BRAF_V600K/R/M | TAGGTGATTTTGGTCTAGCTTCAA |
| 36 | 5BRAF_V600E/D | GGTGATTTTGGTCTAGCTAGAGA |
| 37 | 5KRAS_G12C_1 | AACTTGTGGTAGTTGGAGCTT |
| 38 | 5KRAS_G12V_2 | AACTTGTGGTAGTTGGAGGTGT |
| 39 | 5KRAS_G12D_1 | AACTTGTGGTAGTTGGAGGTGA |
| 40 | 5HER2_T2_9 | GGAAGCATACGTGATGGCATAC |
| 41 | 5HER2_T3_2 | GGAAGCATACGTGATGGCTTAC |
| 42 | 5HER2_T6_3 | GGCTGGTGTGGGCTCCCCGG |
| 43 | **5EGFR_T68_8** | GAACCTCTAAGTCAAGAGCCATCTGT |
| 44 | 3EGFR_G719_1 | GCCTGTGCCAGGGACCT |
| 45 | 3EGFR_T1_1 | CCCCACACAGCAAAGCAGAA |
| 46 | 3EGFR_S768I_1 | GTGGAGGTGAGGCAGATGC |
| 47 | 3EGFR_C797S_1 | CACACACCAGTTGAGCAGGTACT |
| 48 | 3EGFR_T48_6 | CTTACTTTGCCTCCTTCTGC |
| 49 | 3BRAF_V600_1 | CACAAAATGGATCCAGACAACTG |
| 50 | 3KRAS_G12C/V/D_1 | CATATTCGTCCACAAAATGATTCTG |
| 51 | 3HER2_T1_7 | GCTGCACCGTGGATGTCA |
| 52 | **3EGFR_T68_8** | GACATACCTGGAAAAATGGAACC |
| 53 | 5EGFR_G719_MP3_MHS1_M1 | CCGATCTACGTCGAGCGCGTTCGGCACGGTGTATAAGGTAAG GT/3Phos/ |
| 54 | 5EGFR_Ex19del_MP1_MHS2_M1 | GCGAGTCTTACGCTCGGACAAGGAAATCCTCGATGTGAGTTT CTG/3Phos/ |
| 55 | 5EGFR_S768I_MP1_MHS5_M2 | GTAGATGCATAGCCTACCGGACAACCCCCACGTGTGCCG/3P hos/ |
| 56 | 5EGFR_C797S_MP4_MHS8_M4 | TGGGGAGTTACTGTACCGATGGACTATGTCCGGGAACACAAA GACA/3Phos/ |
| 57 | 5EGFR_L858R_MP1_MHS9_M4 | GCAACATCAGATATCGCGTGCGGAAGAGAAAGAATACCATGC AGAA/3Phos/ |
| 58 | 5BRAF_V600_MP2_MHS12_M2 | CGAGCTAACTACGGTACGTCTCGATGGAGTGGGTCCCATCAG TTT/3Phos/ |
| 59 | 5KRASG12_MP1_MHS19_M1 | GTGTCACTCGATTACGCGCAAGAGTGCCTTGACGATACAGCT A/3Phos/ |
| 60 | 5HER2_MP1_MHS14_M3 | CGGAGGACTGTCGCGTATATGCCCAGAAGGCGGGAGACATA/ 3Phos/ |
| 61 | **5KIF11_MP1_MHS15_M2** | GTACTGAGTCGCCGAATCTGGTGTGGATTGTTCATCAATTGG CG/3Phos/ |

Each mediator sequence (or "free mediator" comprising or consisting of a unique generic sequence tag) complementary to exactly one of the generic reporter probes (*i.e.* complementary to the unique generic sequence tag binding site of the generic reporter molecule) as spotted in the amplification chambers of the generic cartridge prototype, was covalently coupled to a target specific sequence to together form one of the 9 mediator probes. The mediator sequences can e.g. be designed as nullomers (*i.e.* a scrambled sequence that does not occur in the human genome) of 10-30 nucleotides in length, but also non-nullomers can be considered. The lengths of the mediator sequences can vary but are considered to be in an acceptable range if they are predicted to give a specific signal by interacting with their corresponding generic reporter and to not cross-react with other generic reporters. Examples of such mediator sequences can be found in Wadle et al. (2015, Biomolecular Detection and Quantification 7:1-8, Real-time PCR probe optimization using design of experiments approach). Sequences as used herein as mediator sequences can also be obtained from Biocartis upon request.

For verifying if the 61-plex with mediator chemistry is suitable to generate target-specific signals in the generic cartridge prototype, the following components were added together into the lysis chamber of each prototype cartridge via its sample entry port:
(i) 27 ul of the mix of the 61 target-specific oligonucleotide subsets comprising primer pairs and mediator probes;
(ii) a mock sample material selected from an FFPE slice, genomic DNA extract, or milliQ water corresponding to no-sample control;
(iii) synthetic target containing a mutation of interest (10-50-100 copies per PCR chamber) or no synthetic target (negative control).

The generic prototype cartridges were then inserted into the Idylla^{™} instruments and the fully automated tests were initiated. During these tests, the Idylla^{™} platform executes pumping of the sample preparation buffer into the lysis chamber and applies heating and high frequency ultrasound (HiFU) treatment to the contents of the lysis chamber in order to obtain a homogenous liquefact. In a next step, the liquefact is heat-inactivated by slow pumping through a heated area followed by transfer of a portion of the heat-inactivated liquefact comprising nucleic acid targets and the mix of target-specific oligonucleotide subsets to each one of the 5 parallel amplification chambers. At this point, the PCR cycling protocol adapted for the 61-plex with the oligonucleotide mix is started, which in the presented herein setting results in generation of free mediators if appropriate target genes or alleles are present in the given liquefact portion. The free mediators hybridize to their respective complementary general reporter probes as spotted in the amplification chambers. The hybridization events result in the generation of fluorescence signals that are measured during the annealing/elongation steps, followed by standard post-processing to correct the data for offset and drift, and for the determination of the first cycle at which amplification can be detected. A Decision Tree with 2 simple parameters was used to remove curves that either had a fluorescence signal that was too low (cf. ratio of signal at the plateau versus at the baseline should be >0.1), or had a Cq that was not in the expected range (cf. 15<Cq<36).

To test the sensitivity, the above procedure was performed using a synthetic target at titration series of 0-10-50-100 copies per PCR chamber (corresponding to 0.0-0.1-0.7-1.4% mutant copies when used in a sample containing 7000 genomic copies per PCR chamber). The synthetic targets were admixed with either a high-background clinical sample containing 7000 genomic copies per PCR chamber or 1000 copies of genomic DNA per PCR chamber, both confirmed upfront to be negative for the mutations of interest. Exemplary obtained amplification curves are shown in Figs.4 and 5.

The sequences of the amplification primers, mediator probes, and generic reporters used in the above-described example are provided in Table 3.

**Table 3**

| SEQ ID NO | oligo name | 5>3 sequence |
|---|---|---|
| 40 | 5HER2_T2_9 | GGAAGCATACGTGATGGCATAC |
| 51 | 3HER2_T1_7 | GCTGCACCGTGGATGTCA |
| 60 | 5HER2_MP1_MHS14_M3 | CGGAGGACTGTCGCGTATATGCCCAGAAGGCGGGAGACATA/3Phos/ |

Firstly, the data unexpectedly shows that with the presented herein methodology, it is possible to detect down to 10 copies per PCR for a selected marker such as HER2 ex20ins, KRAS G12C and EGFR S768I. The method is consequently surprisingly sensitive, given the multiplexing complexity. Furthermore, as no false-positives were detected across over 50 prototype cartridges tested, it can be concluded that the present method meets stringent specificity criteria as well.

Further, the data shows that it is possible to detect one specific marker in a specific chamber, even though the marker was amplified across all of the amplification chambers in parallel identical multiplex reactions with primer pairs and mediator probes provided together with the biological sample. For example, EGFR G719A is detected in channel 2 from chamber A, but not in the other chambers; EGFR S768I is detected in channel 2 from chamber B, but not in the other chambers. Similarly, e.g. EGFR L858R is detected in channel 1 from chamber C, but not in the other chambers; KRAS G12C is detected in channel 1 from chamber D, but not in the other chambers. The data further shows that it is possible to discriminate between two mutants within the same chamber. Namely, C797S is detected in channel 4 from chamber B, and not in the other channels from chamber B; EGFR S768I is detected in channel 2 from chamber B, and not in the other channels from chamber B. Lastly, the data also importantly shows that it is possible to detect multiple markers at the same time in a single amplification chamber using different generic reporters. To illustrate this, each discussed herein target was always being detected in combination with the positive control genomic reference gene, which was used as a sample processing control in all amplification chambers.

In some instances, it may be valuable to discriminate neighboring mutations, as they may lead to a different clinical action. An example of this includes the need to discriminate KRAS G12C from other KRAS G12 or G13 mutations, if it is desired to use the mutation assay for the detection of KRAS mutant tumors that could respond to a specific KRAS G12C-targeting therapy. Traditionally, in a sample-to-result platform with multiple chambers, this is arranged by spotting the ARMS primer for KRAS G12C amplification in a different chamber than the ARMS primers for the other KRAS G12 or G13 mutations. However, when working in a situation where all target-specific oligonucleotides are added together through the sample entry port, it is not possible to keep the spatial separation between the particular ARMS primers. We therefore further modified the ARMS primers to include a stem-loop structure, in which the stem can optionally be composed of the target sequence. We further modified the mediator probe in such a way that it at least partially overlaps with the modified ARMS primer, while in the standard mediator probe design the probes are always downstream of the primer used for amplification. The concept is shown in Fig. 6 for a situation where the binding site of the mediator probe falls completely within the ARMS primer, but it can be envisioned that part of the binding site of the mediator probe falls outside of the ARMS primer. The 3' component of the stem of the ARMS primer can have a sequence that is derived from the target sequence, or can be another sequence. When the target is not amplified, the mediator probe cannot bind to the target sequence and hence cannot create a signal. The mediator probe also cannot bind to its complementary sequence within the ARMS primer, which is inside of the stem-loop configuration and hence inaccessible for the mediator probe. When the target is amplified, the stem structure can be unfolded by the polymerase, thereby creating a binding site for the mediator probe. Once bound, the free mediator is released as the other primer is extended, and the free mediator can bind to the spotted generic reporter and create a signal.

The results are shown in Figure 7, indicating the robustness of the concept as well as the ubiquitously usefulness, *i.e.* the concept is not confined to the generic cartridges of the present invention but can be broadly used in multiplex amplification reactions.

### 4. Development of quality control (QC) amplification using KIF11 for DNA fragmentation assessment

For certain specific applications, like determination of nucleic acid fragmentation or assessment of contamination of short cell-free DNA from plasma with genomic DNA from white blood cells, we have initially envisaged a quality control triplex based on three different housekeeping genes including ABCB, RNaseP, and TFRC. However, having observed that these and other tested genes did not exhibit satisfactory level of stability, and after having identified KIF11 as a surprisingly mutation-free and stable genomic region, we have redeveloped the initial triplex fragmentation assay based on different exons of KIF11. The switch to a single stable region was hypothesized as beneficial to avoid copy number variations, which could occur if several different reference genes were used. Consequently, different exons of KIF11 were used to develop primers and probes for amplification and detection, respectively, of three amplicons of discernably different lengths being 62 bp, 98 bp, and 136 bp. The sequences of the primers and probes as well as conditions of cycling inside of Idylla^{™} cartridges can be obtained from Biocartis upon request. The QC triplex was tested on samples with different degree of DNA fragmentation, including DNA derived from FFPE samples, nucleosomal DNA from blood, intact genomic DNA, etc. As shown in Figure 8, the KIF11-based QC triplex gives a reliable indication of the degree of fragmentation, and hence the quality of the DNA present in a particular sample.

The sequences of the amplification primers, mediator probes, and generic reporters used in the above-described example are provided in Table 4.

**Table 4**

| ***62bp- KIF11*** | non-coding sequence after KIF11 gene | SEQ:* |
|---|---|---|
| FW primer: | CGGGAAGTCAGACGGGTCA | 62 |
| Rev primer: | GAGAGGTACCGGCAGGAGCA | 63 |
| Probe: | CCCCAGACCCCGGCTGCAGCGC | 74 |

| ***98bp- KIF11*** | intron 6 | |
|---|---|---|
| FW primer: | GGAAGCCCAGGATAAAATGTGGCT | 64 |
| Rev primer: | AAGTGCCTCAGGGACCCCTTCA | 65 |
| Probe: | CTAAGGAGGTAAGCCTCAGAGCGTCCCATTCC | 75 |

| ***136bp- KIF11*** | intron 6 | |
|---|---|---|
| FW primer: | GCCATAGTCTCTTCCCTAGCCCCAT | 66 |
| Rev primer: | GCCAATTACCAATGACCCCTCCTT | 67 |
| Probe: | CGTGACCCACATACCCTGACCCACCCC | 76 |

| ***89bp- KIF11*** | Exon 21- Intron 21 boundary | |
|---|---|---|
| FW primer: | GAACCTCTAAGTCAAGAGCCATCTGT | 68 |
| Rev primer: | GACATACCTGGAAAAATGGAACC | 69 |
| Mediator Probe: | ACTGAGTCGCCGAATCGCTGGTGTGGATTGTTCATCAATTGGCGG/3Pho s/ | 77 |
| Universal reporter: | /5Quasar670/CAGCCGGCCAAGACGCGCCGGCT[T(BHQ2)]GGCGAT TCGGCGACTCAGTACTATCAG /3SpC3/ | 80 |

| ***204bp- KIF11*** | Exon 8 | |
|---|---|---|
| FW primer: | GCCGTTCTGGAGCTGTTGATA | 70 |
| Rev primer: | GAGAGATGCAGGAGAAATTGTTGC | 71 |
| Mediator Probe: | CTCGTCTGCTTGTACACTGA CTCGGGAAGCTGGAAATATAAATCAATC/3Phos/ | 78 |
| Universal reporter: | /5Quasar705/CAGCCGGCCAAGACGCGCCGGCT[T(BHQ-2)]GGGATCAGTGTACAAGCAGACGAGAA /3SpC3/ | 81 |

| ***82bp- KIF11*** | Exon 21- Intron 21 boundary | |
|---|---|---|
| Fw primer: | TCTAAGTCAAGAGCCATCTGTA | 72 |
| Rev primer: | GATATGACATACCTGGAAAAATGGAA | 73 |
| Probe: | /5ATTO532N/ACCCCGCCAATTGATGAACAATCCA/3BHQ-1/ | 79 |

| | | |
|---|---|---|
| SEQ:* - SEQ ID NO: | | |

### 5. Determination of DNA fragmentation using KIF11 in mediator chemistry-based qPCR

The next step was the development of DNA fragmentation control qPCR amplification based on KIF11 and its detection with the mediator chemistry readout adapted for the prototype generic cartridge as described above. To do so, a short (82 bp) and a long (204 bp) PCR amplicon targeting different exons of the KIF11 were designed together with two corresponding to them mediator probes. Each of the mediator probes was designed to target its specific generic reporter conjugated with different light-emitting dyes based in channel 5 and channel 1, respectively. Both of the reporters were provided in the same amplification chamber of each prototype cartridge, such that qPCR signals associated with the two amplicons and mediators could be detected in the same reaction. Determination of the Cq difference (delta Cq) between the long and short amplicon qPCR curves was used as a measure of DNA fragmentation. Performance of the "KIF11 DNA fragmentation duplex" was determined on a set of clinical FFPE samples, of which the DNA fragmentation level was previously determined using an orthogonal ddPCR-based method. As a positive control, the assay was also tested on high quality genomic DNA (gDNA) derived from white blood cells (Promega), which is expected to be unfragmented. The results are shown in Figure 9, wherein the lines with circles represent the short amplicon and the regular lines represent the long amplicon. Cq values were determined using a threshold (horizontal line). The results clearly show that the delta Cq value between the long and short KIF11 amplicons as detected by the mediator chemistry, strongly correlates with the degree of DNA fragmentation in the evaluated FFPE samples and that the developed herein fragmentation duplex is readily usable for generic cartridges of the presented herein concept. Such duplex is very well suited to not only provide a positive amplification control for personalized oligonucleotide panels, but, thanks to its ability to detect fragmented samples, it also can provide an indication of the reliability of the final results in function of the sample quality. Then, as extensive fragmentation in FFPE samples correlates with deamination artefacts, due to both being caused by formalin fixation, the present KIF11 duplex assay may also be suitable for prediction of deamination artefacts. Lastly, for cfDNA-based assays from plasma, like the ones targeting circulating foetal, tumor, or organ donor DNA-target panels, the presented here fragmentation assays are likely also suitable to provide information on e.g. contamination with genomic DNA from white blood cells in a plasma sample.

In conclusion, we provided herein a working proof-of-principle demonstration of a highly unusual approach to a fast-tract and extremely versatile diagnostic assay and product development. We believe that the presented herein concepts have an enormous potential to open an entire new venue for rapid, customized, and individualized molecular testing strategies as it eliminates the need for the design, manufacturing and QC release of customized cartridges. In particular, the disclosed herein methods, kits, kits of parts, and uses, can substantially reduce the development costs and the time for bringing new assays to patients. Especially now, such development acceleration is extremely called for, as shown by a sudden outbreak of a global pandemic in 2020, which stalled and delayed production of essential medical and diagnostic products worldwide, delaying treatment decisions and access to adequate medical care for many in need.

## Claims

1. A method for detecting multiple genetic targets, the method comprising:
- providing a mix of multiple oligonucleotide subsets, each of said subsets being specific to a genetic target and comprising a unique to said subset generic sequence tag, ,
wherein each of said subsets is adapted to generate, under nucleic acid amplification conditions and in the presence of the genetic target, a detectable nucleic acid product comprising the unique generic sequence tag;
- separately from the mix, providing a cartridge comprising
(i) an entry port for accepting a biological sample,
(ii) a nucleic acid isolation compartment positioned downstream of the entry port,
(iii) reagents for nucleic acid amplification;
(iv) one or more nucleic acid amplification compartments positioned downstream of the nucleic acid extraction chamber, and
(v) a plurality of generic reporters, wherein each one of the plurality of generic reporters comprises a generic sequence specific to the unique generic sequence tag comprised in one of the detectable nucleic acid products and is adapted to generate a signal in the presence of said detectable nucleic acid product;
wherein a biological sample and the mix are inserted into the cartridge by a user, the method further comprising
- operating the cartridge after the insertion of the biological sample and the mix, wherein the operating comprises performing nucleic acid isolation from the biological sample followed by a multiplex nucleic acid amplification comprising the mix;
- detecting a signal generated from at least one of the plurality of generic reporters comprised inside of the cartridge, if at least one detectable nucleic acid product is generated from said amplification.

2. Method according to claim 1, wherein at least one of the multiple oligonucleotide subsets is specific to a genetic target that was identified in a Next Generation Sequencing (NGS) analysis performed on a sample from an individual from whom the biological sample was obtained.

3. Method according to any of the above claims, wherein the nucleic acids isolated from the biological sample and the mix of multiple oligonucleotide subsets are moved inside of the integrated fluidic cartridge into at least two or more different amplification compartments.

4. Method according claim 3, wherein different generic reporters are provided in the different amplification compartments.

5. Method according to any of the above claims, wherein one or more of the multiple oligonucleotide subsets comprises at least one primer and at least one mediator probe, said mediator probe comprising the unique generic sequence tag, and wherein the detectable nucleic acid product is a cleaved free mediator comprising said unique generic sequence tag.

6. Method according to claim 5, wherein the at least one primer is an ARMS primer.

7. Method according to claim 6, wherein the ARMS primer comprises a stem-loop structure, and preferably wherein the mediator probe sequence at least partially overlaps with the sequence comprised in said stem-loop structure.

8. Method according to any of the above claims, wherein the nucleic acid isolation from the biological sample is performed in the presence of the mix of multiple oligonucleotide subsets.

9. Method according to any of the above claims, wherein at least a part of the oligonucleotide subsets is designed by a computer-implemented method comprising machine learning

10. Method according to any of the above claims, wherein one of the multiple oligonucleotide subsets comprises a primer specific to a region in human KIF11 gene, wherein a KIF11 amplicon generated with said primer is used as a genomic reference gene.

11. A kit comprising, provided as separate components:
- a mix of multiple oligonucleotide subsets, each of said subsets being specific to a genetic target and comprising a unique to said subset unique generic sequence tag, defined as a sequence not present in the genetic information of the organism from which the genetic targets are being detected,
wherein each of said subsets is adapted to generate, under nucleic acid amplification conditions and in the presence of the genetic target, a detectable nucleic acid product comprising the unique generic sequence tag; and
- an integrated fluidic cartridge comprising
(i) an entry port for accepting a biological sample;
(ii) a nucleic acid isolation compartment positioned downstream of the entry port;
(iii) reagents for nucleic acid amplification;
(iv) one or more nucleic acid amplification compartments positioned downstream of the nucleic acid isolation compartment, and
(v) a plurality of generic reporters, wherein each of the plurality of generic reporters comprises a generic sequence specific to one of the unique generic sequence tags and is adapted to generate a signal in the presence of the detectable nucleic acid product comprising the unique generic sequence tag.

12. The kit according to claim 11, wherein an oligonucleotide subset from the mix of multiple oligonucleotide subsets comprises at least a primer and a mediator probe, and preferably wherein the primer is an ARMS primer.

13. The kit according to claim 12, wherein the ARMS primer comprises a stem-loop structure and wherein the mediator probe sequence at least partially overlaps with the sequence comprised in said stem-loop structure.

14. The kit according to any of claims 11-13, wherein one or more of the multiple oligonucleotide subsets comprises a primer specific to a region in human KIF11 gene, preferably wherein at least two of the multiple oligonucleotide subsets comprise primers specific to different regions in human KIF11 gene, wherein said primers are designed to generate two KIF11 amplicons of discernably different lengths.

15. Use of the method of claims 1-10 or of kits according to claims 11-14 for detecting multiple genetic targets, preferably in a sample obtained from a cancer patient, more preferably as part of the patient's surveillance following NGS analysis or in minimal residual disease monitoring.


SEQUENCE LISTING

<110>  Biocartis NV

<120>  GENERIC CARTRIDGE AND METHOD FOR MULTIPLEX NUCLEIC ACID DETECTION

<130>  A-2001-EP-02_NLO-P6106765EP

<150>  EP20204806
<151>  2020-10-29

<150>  EP20214901
<151>  2020-12-17

<160>  81

<170>  PatentIn version 3.5

<210>  1
<211>  19
<212>  DNA
<213>  synthetic DNA

<400>  1
aaaagatcaa agtgctggc                                                    19


<210>  2
<211>  25
<212>  DNA
<213>  synthetic DNA

<400>  2
gaattcaaaa agatcaaagt gcaga                                             25


<210>  3
<211>  24
<212>  DNA
<213>  synthetic DNA

<400>  3
aattcaaaaa gatcaaagtg cggt                                              24


<210>  4
<211>  23
<212>  DNA
<213>  synthetic DNA

<400>  4
ctcttgagga tcttgaagga tga                                               23


<210>  5
<211>  22
<212>  DNA
<213>  synthetic DNA

<400>  5
gctctcttga ggatcttgta ga                                                22

<210>  6
<211>  20
<212>  DNA
<213>  synthetic DNA

<400>  6
ctcttgagga tcttgatggc                                                    20


<210>  7
<211>  23
<212>  DNA
<213>  synthetic DNA

<400>  7
ctcttgagga tcttgaagca tac                                                23


<210>  8
<211>  20
<212>  DNA
<213>  synthetic DNA

<400>  8
ctcttgagga tcttgatggg                                                    20


<210>  9
<211>  21
<212>  DNA
<213>  synthetic DNA

<400>  9
tctcttgagg atcttgatgg t                                                  21


<210>  10
<211>  21
<212>  DNA
<213>  synthetic DNA

<400>  10
attcccgtcg ctatcaaaac a                                                  21


<210>  11
<211>  20
<212>  DNA
<213>  synthetic DNA

<400>  11
ccgtcgctat caagacatct                                                    20


<210>  12
<211>  22
<212>  DNA
<213>  synthetic DNA

<400>  12
ccgtcgctat caaggaatcg aa                                                 22

<210>  13
<211>  20
<212>  DNA
<213>  synthetic DNA

<400>  13
cgtcgctatc aaggaatctc                                                    20


<210>  14
<211>  19
<212>  DNA
<213>  synthetic DNA

<400>  14
tcgctatcaa ggaaccaac                                                     19


<210>  15
<211>  19
<212>  DNA
<213>  synthetic DNA

<400>  15
cgtcgctatc aaggttccg                                                     19


<210>  16
<211>  19
<212>  DNA
<213>  synthetic DNA

<400>  16
ccgtcgctat caaggcatc                                                     19


<210>  17
<211>  20
<212>  DNA
<213>  synthetic DNA

<400>  17
gtcgctatca aggaaccgaa                                                    20


<210>  18
<211>  21
<212>  DNA
<213>  synthetic DNA

<400>  18
cgtcgctatc aaggaaccat c                                                  21


<210>  19
<211>  19
<212>  DNA
<213>  synthetic DNA

<400>  19
cgtcgctatc aaggaagca                                                     19

<210>  20
<211>  21
<212>  DNA
<213>  synthetic DNA

<400>  20
cgtcgctatc aaggagccaa c                                                21


<210>  21
<211>  21
<212>  DNA
<213>  synthetic DNA

<400>  21
cgtcgctatc aaggaatcat c                                                21


<210>  22
<211>  22
<212>  DNA
<213>  synthetic DNA

<400>  22
tcccgtcgct atcaaaatat ct                                               22


<210>  23
<211>  19
<212>  DNA
<213>  synthetic DNA

<400>  23
tcccgtcgct atcaagtct                                                   19


<210>  24
<211>  20
<212>  DNA
<213>  synthetic DNA

<400>  24
gtcgctatca aggaacagaa                                                  20


<210>  25
<211>  19
<212>  DNA
<213>  synthetic DNA

<400>  25
ccgtcgctat caaggctcc                                                   19


<210>  26
<211>  19
<212>  DNA
<213>  synthetic DNA

<400>  26
cgtcgctatc aaggatccg                                                   19

<210>  27
<211>  19
<212>  DNA
<213>  synthetic DNA

<400>  27
gtcgctatca aggagcaat                                                    19


<210>  28
<211>  20
<212>  DNA
<213>  synthetic DNA

<400>  28
gaagcctacg tgatgggcat                                                   20


<210>  29
<211>  20
<212>  DNA
<213>  synthetic DNA

<400>  29
ctccaggaag ccttccagga                                                   20


<210>  30
<211>  17
<212>  DNA
<213>  synthetic DNA

<400>  30
ctcatgccct tcggctc                                                      17


<210>  31
<211>  17
<212>  DNA
<213>  synthetic DNA

<400>  31
gctcatgccc ttcggca                                                      17


<210>  32
<211>  23
<212>  DNA
<213>  synthetic DNA

<400>  32
gtcaagatca cagattttgg tcg                                               23


<210>  33
<211>  24
<212>  DNA
<213>  synthetic DNA

<400>  33
gtcaagatca cagattttgg gcgt                                              24

<210>  34
<211>  20
<212>  DNA
<213>  synthetic DNA

<400>  34
gattttgggc tggccaaaca                                                    20


<210>  35
<211>  24
<212>  DNA
<213>  synthetic DNA

<400>  35
taggtgattt tggtctagct tcaa                                               24


<210>  36
<211>  23
<212>  DNA
<213>  synthetic DNA

<400>  36
ggtgattttg gtctagctag aga                                                23


<210>  37
<211>  21
<212>  DNA
<213>  synthetic DNA

<400>  37
aacttgtggt agttggagct t                                                  21


<210>  38
<211>  22
<212>  DNA
<213>  synthetic DNA

<400>  38
aacttgtggt agttggaggt gt                                                 22


<210>  39
<211>  22
<212>  DNA
<213>  synthetic DNA

<400>  39
aacttgtggt agttggaggt ga                                                 22


<210>  40
<211>  22
<212>  DNA
<213>  synthetic DNA

<400>  40
ggaagcatac gtgatggcat ac                                                 22

<210>  41
<211>  22
<212>  DNA
<213>  synthetic DNA

<400>  41
ggaagcatac gtgatggctt ac                                                22


<210>  42
<211>  20
<212>  DNA
<213>  synthetic DNA

<400>  42
ggctggtgtg ggctccccgg                                                   20


<210>  43
<211>  26
<212>  DNA
<213>  synthetic DNA

<400>  43
gaacctctaa gtcaagagcc atctgt                                            26


<210>  44
<211>  17
<212>  DNA
<213>  synthetic DNA

<400>  44
gcctgtgcca gggacct                                                      17


<210>  45
<211>  20
<212>  DNA
<213>  synthetic DNA

<400>  45
ccccacacag caaagcagaa                                                   20


<210>  46
<211>  19
<212>  DNA
<213>  synthetic DNA

<400>  46
gtggaggtga ggcagatgc                                                    19


<210>  47
<211>  23
<212>  DNA
<213>  synthetic DNA

<400>  47
cacacaccag ttgagcaggt act                                               23

<210>  48
<211>  20
<212>  DNA
<213>  synthetic DNA

<400>  48
cttactttgc ctccttctgc                                                    20


<210>  49
<211>  23
<212>  DNA
<213>  synthetic DNA

<400>  49
cacaaaatgg atccagacaa ctg                                                23


<210>  50
<211>  25
<212>  DNA
<213>  synthetic DNA

<400>  50
catattcgtc cacaaaatga ttctg                                              25


<210>  51
<211>  18
<212>  DNA
<213>  synthetic DNA

<400>  51
gctgcaccgt ggatgtca                                                      18


<210>  52
<211>  23
<212>  DNA
<213>  synthetic DNA

<400>  52
gacatacctg gaaaaatgga acc                                                23


<210>  53
<211>  44
<212>  DNA
<213>  synthetic DNA

<400>  53
ccgatctacg tcgagcgcgt tcggcacggt gtataaggta aggt                         44


<210>  54
<211>  45
<212>  DNA
<213>  synthetic DNA

<400>  54
gcgagtctta cgctcggaca aggaaatcct cgatgtgagt ttctg                        45

<210>  55
<211>  39
<212>  DNA
<213>  synthetic DNA

<400>  55
gtagatgcat agcctaccgg acaaccccca cgtgtgccg                                39


<210>  56
<211>  46
<212>  DNA
<213>  synthetic DNA

<400>  56
tggggagtta ctgtaccgat ggactatgtc cgggaacaca aagaca                        46


<210>  57
<211>  46
<212>  DNA
<213>  synthetic DNA

<400>  57
gcaacatcag atatcgcgtg cggaagagaa agaataccat gcagaa                        46


<210>  58
<211>  45
<212>  DNA
<213>  synthetic DNA

<400>  58
cgagctaact acggtacgtc tcgatggagt gggtcccatc agttt                         45


<210>  59
<211>  43
<212>  DNA
<213>  synthetic DNA

<400>  59
gtgtcactcg attacgcgca agagtgcctt gacgatacag cta                           43


<210>  60
<211>  41
<212>  DNA
<213>  synthetic DNA

<400>  60
cggaggactg tcgcgtatat gcccagaagg cgggagacat a                             41


<210>  61
<211>  44
<212>  DNA
<213>  synthetic DNA

<400>  61
gtactgagtc gccgaatctg gtgtggattg ttcatcaatt ggcg                          44

<210>  62
<211>  19
<212>  DNA
<213>  synthetic DNA

<400>  62
cgggaagtca gacgggtca                                                    19


<210>  63
<211>  20
<212>  DNA
<213>  synthetic DNA

<400>  63
gagaggtacc ggcaggagca                                                   20


<210>  64
<211>  24
<212>  DNA
<213>  synthetic DNA

<400>  64
ggaagcccag gataaaatgt ggct                                              24


<210>  65
<211>  22
<212>  DNA
<213>  synthetic DNA

<400>  65
aagtgcctca gggacccctt ca                                                22


<210>  66
<211>  25
<212>  DNA
<213>  synthetic DNA

<400>  66
gccatagtct cttccctagc cccat                                             25


<210>  67
<211>  24
<212>  DNA
<213>  synthetic DNA

<400>  67
gccaattacc aatgacccct cctt                                              24


<210>  68
<211>  26
<212>  DNA
<213>  synthetic DNA

<400>  68
gaacctctaa gtcaagagcc atctgt                                            26

<210>  69
<211>  23
<212>  DNA
<213>  synthetic DNA

<400>  69
gacatacctg gaaaaatgga acc                                              23


<210>  70
<211>  21
<212>  DNA
<213>  synthetic DNA

<400>  70
gccgttctgg agctgttgat a                                                21


<210>  71
<211>  24
<212>  DNA
<213>  synthetic DNA

<400>  71
gagagatgca ggagaaattg ttgc                                             24


<210>  72
<211>  22
<212>  DNA
<213>  synthetic DNA

<400>  72
tctaagtcaa gagccatctg ta                                               22


<210>  73
<211>  26
<212>  DNA
<213>  synthetic DNA

<400>  73
gatatgacat acctggaaaa atggaa                                           26


<210>  74
<211>  22
<212>  DNA
<213>  synthetic DNA

<400>  74
ccccagaccc cggctgcagc gc                                               22


<210>  75
<211>  32
<212>  DNA
<213>  synthetic DNA

<400>  75
ctaaggaggt aagcctcaga gcgtcccatt cc                                    32

<210>  76
<211>  27
<212>  DNA
<213>  synthetic DNA

<400>  76
cgtgacccac ataccctgac ccacccc                                          27


<210>  77
<211>  45
<212>  DNA
<213>  synthetic DNA

<400>  77
actgagtcgc cgaatcgctg gtgtggattg ttcatcaatt ggcgg                      45


<210>  78
<211>  48
<212>  DNA
<213>  synthetic DNA

<400>  78
ctcgtctgct tgtacactga ctcgggaagc tggaaatata aatcaatc                   48


<210>  79
<211>  25
<212>  DNA
<213>  synthetic DNA

<400>  79
accccgccaa ttgatgaaca atcca                                            25


<210>  80
<211>  51
<212>  DNA
<213>  synthetic DNA

<400>  80
cagccggcca agacgcgccg gcttggcgat tcggcgactc agtactatca g               51


<210>  81
<211>  50
<212>  DNA
<213>  synthetic DNA

<400>  81
cagccggcca agacgcgccg gcttgggatc agtgtacaag cagacgagaa                 50